(19) Europäisches Patentamt
European Patent Office
Office européen des brevets

(11) EP 4 268 796 A1

# (12) EUROPEAN PATENT APPLICATION

published in accordance with Art. 153(4) EPC

(43) Date of publication:
**01.11.2023 Bulletin 2023/44**

(21) Application number: **21911110.1**

(22) Date of filing: **24.12.2021**

(51) International Patent Classification (IPC):
***A61K 6/887*** *(2020.01)* ***A61C 13/15*** *(2006.01)*

(52) Cooperative Patent Classification (CPC):
**A61C 19/003; A61K 6/887**

(86) International application number:
**PCT/JP2021/048424**

(87) International publication number:
**WO 2022/138973 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.12.2020 JP 2020218033**

(71) Applicant: **Kuraray Noritake Dental Inc.**
**Kurashiki-shi, Okayama 710-0801 (JP)**

(72) Inventor: **MATSUURA, Ryo**
**Tainai-shi, Niigata 959-2653 (JP)**

(74) Representative: **D Young & Co LLP**
**120 Holborn**
**London EC1N 2DY (GB)**

## (54) DENTAL COMPOSITION

(57) An object of the present invention is to provide a dental composition that exhibits small polymerization shrinkage stress, and provides excellent mechanical strength. The present invention relates to a dental composition comprising a compound (A), a monomer (B) having a solubility of 50 g/L or more in acetone at 25°C, and a polymerization initiator (C),
wherein the compound (A) has a weight-average molecular weight of 1,000 to 80,000, and includes a Tg of less than 40°C, in addition to having a solubility of less than 50 g/L in acetone at 25°C. Preferably, the compound (A) comprises a compound having a polymerizable group.

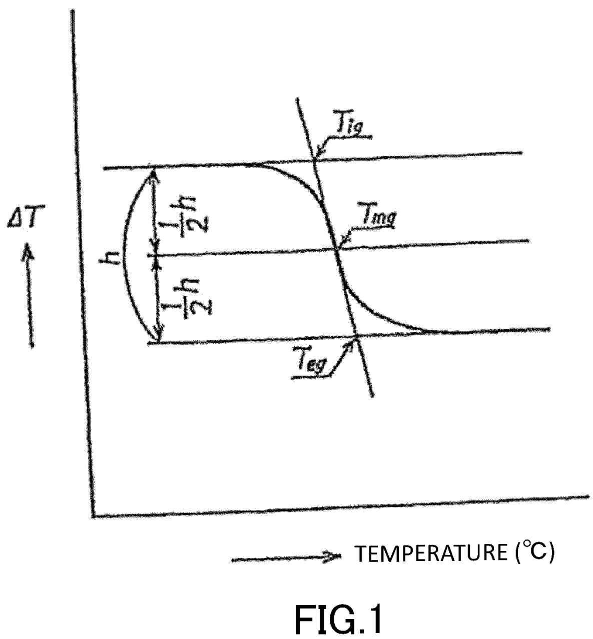


FIG.1

EP 4 268 796 A1

## Description

TECHNICAL FIELD

The present invention relates to a dental composition used in the field of dental treatment.

BACKGROUND ART

Restorations using dental bonding materials and dental composite resins have been generally practiced for the treatment of dental caries and associated defects. The restorative treatment typically follows the following procedure. First, the damaged portion including the cavity is removed to create a pit, and a dental bonding material is applied to the pit. The dental bonding material is then cured by irradiating the applied area with visible light. Next, a dental composite resin is filled over the cured dental bonding material layer, and the dental composite resin is cured with visible light.

Dental composite resins have become widely used as alternative materials of conventional metallic materials because of excellent aesthetics comparable to natural teeth, and good ease of handling. A dental composite resin is typically composed mainly of a polymerizable monomer, a polymerization initiator, and a filler. In view of body's safety and the mechanical strength and wear resistance of the cured product, radical polymerizable polyfunctional (meth)acrylates are commonly used as polymerizable monomers. Particularly common are 2,2-bis[4-(3-methacryloyloxy-2-hydroxypropoxy)phenyl]propane (Bis-GMA), and 2,2,4-trimethylhexamethylenebis(2-carbamoyloxyethyl)dimethacrylate (UDMA). Because Bis-GMA and UDMA are highly viscous on its own, these are typically used by being diluted with a polymerizable monomer having a relatively low viscosity, such as triethylene glycol dimethacrylate (3G).

Although dental composite resins are now widely used in clinical practice, there is a need for improvements in the following areas. Specifically, it has been noted that there is a great deal of room for improvement in areas such as ease of handling of a paste of a polymerizable composition used as a dental composite resin, improvement of the flexural strength, elastic modulus, and wear resistance of a cured product, a reduction of water absorption and discoloration or staining, a reduction of polymerization shrinkage stress during cure, and providing aesthetics comparable to natural teeth.

In recent years, there is particularly a strong need to minimize polymerization shrinkage stress because a large polymerization shrinkage stress leads to a contraction gap, which occurs when the dental composite resin pulls away from the bonded surface. Formation of a contraction gap causes various problems, such as secondary caries, tooth pulp stimulation, staining, and detachment of restorations.

Methods that add a polymer to a dental composition have been proposed as a way of reducing such polymerization shrinkage stress. Patent Literature 1 discloses a dental composition that uses a branched polymer to reduce polymerization shrinkage stress. Patent Literature 2 discloses a dental composition that uses a macrocyclic oligomer to reduce polymerization shrinkage stress. Patent Literature 3 discloses a particulate composite material comprised of an organic binder and an inorganic filler. Patent Literature 4 discloses a dental material formed of a composition comprising a metal fine powder, a (meth)acrylic polymer particle, a polymerizable monomer component, and a polymerization catalyst. Patent Literature 5 discloses a dental filling and restorative kit comprising a transparent external filler comprised of particles having a maximum diameter of 0.5 mm to 4.0 mm, and a dental polymerizable composition containing a radical polymerizable monomer and a photopolymerization initiator.

However, Patent Literature 1 cannot provide a sufficient level of reduction in polymerization shrinkage stress, and is also insufficient in terms of strength because the filling rate of inorganic filler cannot be increased high enough due to an increased viscosity of the composition attributed to the branched polymer having a weight-average molecular weight of 20,000 or more. Patent Literature 2 fails to reduce polymerization shrinkage stress to a sufficient level even with the means it discloses. Patent Literature 2 is also insufficient in terms of strength because the macrocyclic oligomer contained increases viscosity, and the filling rate of inorganic filler cannot be increased to a satisfactory level by a phenomenon similar to that observed in Patent Literature 1. In Patent Literature 3, a particulate composite material of large particle size is contained in large amounts, and this produces roughness in the paste. Additionally, because the particulate composite material is treated with a polymerizable monomer, the polymerization shrinkage stress reducing effect was found to be insufficient after polymerization upon cure. Patent Literature 4 is insufficient in terms of mechanical strength as with Patent Literature 3 because the constituent components of the dental material are mostly (meth)acrylic polymer particles. As with Patent Literature 3, Patent Literature 5 is also insufficient in terms of mechanical strength. Additionally, the effect cannot be produced in a reliable fashion because the excessively large particle size leads to some pastes containing particles while others have no particles when a small amount of paste is used in clinical practice.

Other known dental compositions contain an oligomer added to dental compositions (for example, Patent Literatures 6 to 9). However, in Patent Literatures 6 to 9, an oligomer is added with the intention to improve mechanical strength, and the idea of reducing polymerization shrinkage stress is lacking. In addition, studies by the present inventor confirmed that the desired effect of reducing polymerization shrinkage stress was absent.

## CITATION LIST

### Patent Literature

Patent Literature 1: JP 2014-24775 A
Patent Literature 2: JP 2012-188672 A
Patent Literature 3: JP 2002-256010 A
Patent Literature 4: JP H11-29428 A
Patent Literature 5: JP 2016-175851 A
Patent Literature 6: JP S50-042696 A
Patent Literature 7: JP 2006-510583 T
Patent Literature 8: JP 2009-184971 A
Patent Literature 9: JP 2011-144121 A

## SUMMARY OF INVENTION

### Technical Problem

As discussed above, no dental composition is available in related art that exhibits small polymerization shrinkage stress, and provides excellent mechanical strength in the cured product.

It is accordingly an object of the present invention to provide a dental composition that exhibits small polymerization shrinkage stress, and provides excellent mechanical strength in the cured product.

### Solution to Problem

The present inventor conducted intensive studies, and found that the foregoing issues can be solved with a dental composition that comprises a compound having a weight-average molecular weight equal to or above a certain value, and having no greater than a specific level of solubility in acetone. The present invention was completed after further studies.

Specifically, the present invention includes the following.

[1] A dental composition comprising a compound (A), a monomer (B) having a solubility of 50 g/L or more in acetone at 25°C, and a polymerization initiator (C),
wherein the compound (A) has a weight-average molecular weight of 1,000 to 80,000, and includes a Tg of less than 40°C, in addition to having a solubility of less than 50 g/L in acetone at 25°C.
[2] The dental composition according to [1], wherein the compound (A) comprises a compound having a polymerizable group.
[3] The dental composition according to [1] or [2], wherein the content of the compound (A) is 0.1 to 50 parts by mass with respect to total 100 parts by mass of the compound (A) and the monomer (B).
[4] The dental composition according to [2] or [3], wherein the compound having a polymerizable group comprises a urethanized (meth)acrylic compound (A-1) having a urethane bond.
[5] The dental composition according to any one of [1] to [4], wherein the compound (A) has a solubility of less than 20 g/L in acetone at 25°C.
[6] The dental composition according to any one of [1] to [5], wherein the monomer (B) comprises a monomer (B-1) having an acidic group.
[7] The dental composition according to any one of [1] to [6], which further comprises a filler (D).
[8] The dental composition according to any one of [2] to [7], wherein the compound (A) has a weight-average molecular weight of 1,250 or more and less than 20,000 per polymerizable group.
[9] A dental composite resin comprising a dental composition of any one of [1] to [8].
[10] A self-adhesive dental composite resin comprising a dental composition of any one of [1] to [8].
[11] A dental cement comprising a dental composition of any one of [1] to [8].

### Advantageous Effects of Invention

According to the present invention, a dental composition can be provided that exhibits small polymerization shrinkage stress, and provides excellent mechanical strength in the cured product. Because of its characteristics, the

dental composition can be suitably used as, for example, a dental composite resin, a self-adhesive dental composite resin, or a dental cement.

BRIEF DESCRIPTION OF DRAWINGS

FIG. 1 is a schematic view illustrating how a glass transition temperature is determined when glass transition shows a step-like change as in FIG. 3 of JIS K 7121-1987 representing a method of determination of glass transition temperature.

DESCRIPTION OF EMBODIMENTS

A dental composition of the present invention is a dental composition comprising a compound (A) that has a weight-average molecular weight of 1,000 to 80,000, and that includes a Tg of less than 40°C, in addition to having a solubility of less than 50 g/L in acetone at 25°C (hereinafter, also referred to as "compound (A)"); a monomer (B) having a solubility of 50 g/L or more in acetone at 25°C (hereinafter, also referred to as "monomer (B)"); and a polymerization initiator (C).

In the present specification, "(meth)acryl" is a collective term for methacryl and acryl, and the same applies to similar expressions, such as "(meth)acrylic acid" and "(meth)acrylonitrile". In the present specification, the upper limits and lower limits of numeric ranges (for example, ranges of contents of components, ranges of values calculated from components, and ranges of physical properties) can be appropriately combined.

The reason a dental composition of the present invention exhibits small polymerization shrinkage stress during polymerization and cure is unclear. However, the following speculation has been made. Compound (A) and monomer (B) become less compatible with each other as the difference in polarity, represented by the difference in solubility in acetone, between compound (A) and monomer (B) increases. When a composition containing a compound (A) and a monomer (B) having a large polarity difference is cured, the composition segregates into two phases of monomer (B) component and compound (A) component such as by forming an island-in-sea structure. In a dental composition of the present invention, the compound (A) forms a flexible phase because it is a flexible material with a low glass transition temperature. Presumably, the phase formed by compound (A) undergoes deformation during polymerization and cure of the dental composition, and relieves the polymerization shrinkage stress that generates during cure.

The following describes the components used in a dental composition of the present invention.

[Compound (A)]

In the present invention, the compound (A) has a weight-average molecular weight of 1,000 to 80,000, and includes a Tg of less than 40°C, in addition to having a solubility of less than 50 g/L in acetone at 25°C (hereinafter, solubility in acetone at 25°C is also referred to simply as "solubility in acetone"). In a dental composition of the present invention, the compound (A) is used to impart a low polymerization shrinkage stress.

In view of the mechanical strength of a cured product of the dental composition, the compound (A) preferably comprises a compound having a polymerizable group. Examples of the polymerizable group include a vinyl group, a (meth)acryl group, and a (meth)acrylamide group. The polymerizable group is preferably a (meth)acryl group or a (meth)acrylamide group, more preferably a (meth)acryl group. A compound having a weight-average molecular weight of 1,000 or more with no polymerizable group will be described later. The following describes a compound (A) having a (meth)acryl group as a polymerizable group. The compound (A) having a (meth)acryl group can be broadly classified into a urethanized (meth)acrylic compound (A-1) having a urethane skeleton (hereinafter, also referred to as "urethanized (meth)acrylic compound (A-1)"), and a (meth)acrylic compound (A-2) having no urethane skeleton. The urethanized (meth)acrylic compound (A-1) is preferred in view of ease of introducing of a (meth)acryl group, and the effect to reduce polymerization shrinkage stress.

The urethanized (meth)acrylic compound (A-1) can be easily synthesized by, for example, an addition reaction of a polyol containing a polymer skeleton (described later), a compound having an isocyanate group (-NCO), and a (meth)acrylic compound having a hydroxyl group (-OH). Alternatively, the urethanized (meth)acrylic compound (A-1) can be synthesized with ease by, for example, allowing lactone or alkylene oxide to undergo a ring-opening addition reaction with a (meth)acrylic compound having a hydroxyl group, and causing the resulting compound having a terminal hydroxyl group to undergo an addition reaction with a compound having an isocyanate group. The (meth)acrylic compound that donates a (meth)acryl group to a polymer can introduce a (meth)acryl group to, for example, a polymer of a monomer having a hydroxyl group through a dehydrocondensation reaction of (meth)acrylic acid.

· Urethanized (Meth)Acrylic Compound (A-1)

The urethanized (meth)acrylic compound (A-1) is preferably a (meth)acrylate having a structure (a polymer skeleton) selected from the group consisting of a polyester, a polycarbonate, a polyurethane, a polyether, a poly-conjugated diene, and a hydrogenated poly-conjugated diene, in addition to a urethane bond. More preferably, the urethanized (meth)acrylic compound (A-1) is a (meth)acrylate having, within the molecule, a urethane bond, and at least one polyol moiety selected from the group consisting of a polyester, a polycarbonate, a polyurethane, a polyether, a poly-conjugated diene, and a hydrogenated poly-conjugated diene each having a structure derived from a C4 to C18 aliphatic diol unit having a branched structure. These are not particularly limited, as long as the above structure is present.

Examples of the polyester include: a polymer of a dicarboxylic acid (e.g., an aromatic dicarboxylic acid such as phthalic acid or isophthalic acid, or an unsaturated aliphatic dicarboxylic acid such as maleic acid) and an aliphatic diol having 2 to 18 carbon atoms; a polymer of a dicarboxylic acid (e.g., a saturated aliphatic dicarboxylic acid such as adipic acid or sebacic acid) and an aliphatic diol having 2 to 18 carbon atoms; a polymer of β-propiolactone; a polymer of γ-butyrolactone; a polymer of 6-valerolactone; a polymer of ε-caprolactone; and a copolymer of these. Preferred are a polymer of a dicarboxylic acid (an aromatic dicarboxylic acid such as phthalic acid or isophthalic acid, or an unsaturated aliphatic dicarboxylic acid such as maleic acid) and an aliphatic diol having 2 to 12 carbon atoms; and a polymer of a dicarboxylic acid (a saturated aliphatic dicarboxylic acid such as adipic acid or sebacic acid) and an aliphatic diol having 2 to 12 carbon atoms.

Examples of the polycarbonate include a polycarbonate derived from an aliphatic diol having 2 to 18 carbon atoms, a polycarbonate derived from bisphenol A, and a polycarbonate derived from a C2 to C18 aliphatic diol and bisphenol A.
Preferred are a polycarbonate derived from an aliphatic diol having 2 to 12 carbon atoms, a polycarbonate derived from bisphenol A, and a polycarbonate derived from a C2 to C12 aliphatic diol and bisphenol A.

Examples of the polyurethane include a polymer of a C2 to C18 aliphatic diol and a C1 to C18 diisocyanate. Preferred is a polymer of a C2 to C12 aliphatic diol and a C1 to C12 diisocyanate.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these.

Among these structures, a polyester, a polycarbonate, and a poly-conjugated diene are preferred in view of superior flexibility and superior water resistance. A polyol having the polymer skeleton mentioned above can be used for the production of urethanized (meth)acrylic compound (A-1).

Examples of the compound having an isocyanate group include hexamethylene diisocyanate (HDI), tolylene diisocyanate (TDI), xylylene diisocyanate (XDI), diphenylmethane diisocyanate (MDI), isophorone diisocyanate (IPDI), trimethylhexamethylene diisocyanate (TMHMDI), tricyclodecane diisocyanate (TCDDI), and adamantane diisocyanate (ADI).

Examples of the (meth)acrylic compound having a hydroxyl group include:

hydroxy (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis[4-[3-(meth)acryloyloxy-2-hydroxypropoxy]phenyl]propane, 1,2-bis[3-(meth)acryloyloxy-2-hydroxypropoxy]ethane, pentaerythritol tri(meth)acrylate, and tri or tetra(meth)acrylates of dipentaerythritol; and
hydroxy (meth)acrylamide compounds such as N-hydroxyethyl (meth)acrylamide, and N,N-bis(2-hydroxyethyl)(meth)acrylamide.

Examples of the C4 to C18 aliphatic diol having a branched structure include 2-methyl-1,3-propanediol, 2,2-diethyl-1,3-propanediol, 1,3-butanediol, 2-methyl-1,4-butanediol, neopentyl glycol, 3-methyl-1,5-pentanediol, 2-methyl-1,8-octanediol, 2,7-dimethyl-1,8-octanediol, 2-methyl-1,9-nonanediol, 2,8-dimethyl-1,9-nonanediol, 2-methyl-1,10-decanediol, 2,9-dimethyl-1,10-decanediol, 2-methyl-1,11-undecanediol, 2,10-dimethyl-1,11-undecanediol, 2-methyl-1,12-dodecanediol, 2,11-dimethyl-1,12-dodecanediol, 2-methyl-1,13-tridecanediol, 2,12-dimethyl-1,13-tridecanediol, 2-methyl-1,14-tetradecanediol, 2,13-dimethyl-1,14-tetradecanediol, 2-methyl-1,15-pentadecanediol, 2,14-dimethyl-1,15-pentadecanediol, 2-methyl-1,16-hexadecanediol, and 2,15-dimethyl-1,16-hexadecanediol. In view of providing a dental composition having superior curability, the polyol components used are preferably C5 to C12 aliphatic diols having a methyl-group side chain, for example, such as 2-methyl-1,4-butanediol, 3-methyl-1,5-pentanediol, 2-methyl-1,8-octanediol, 2,7-dimethyl-1,8-octanediol, 2-methyl-1,9-nonanediol, and 2,8-dimethyl-1,9-nonanediol. The polyol components

are more preferably 2-methyl-1,4-butanediol, 3-methyl-1,5-pentanediol, 2-methyl-1,8-octanediol, and 2,7-dimethyl-1,8-octanediol, even more preferably 3-methyl-1,5-pentanediol, and 2-methyl-1,8-octanediol.

The addition reaction of the compound having an isocyanate group and the (meth)acrylic compound having a hydroxyl group can be performed following a known method, and the method is not particularly limited.

The urethanized (meth)acrylic compound (A-1) obtained is, for example, a product of a reaction of any combination of: the polyol having at least one structure selected from the group consisting of a polyester, a polycarbonate, a polyurethane, a polyether, a poly-conjugated diene, and a hydrogenated poly-conjugated diene; the compound having an isocyanate group; and the (meth)acrylic compound having a hydroxyl group. The glass transition temperature and acetone solubility of the urethanized (meth)acrylic compound (A-1) can be adjusted by adjusting the skeleton or molecular weight of a structure (polymer skeleton) selected from the group consisting of a polyester, a polycarbonate, a polyurethane, a polyether, a poly-conjugated diene, and a hydrogenated poly-conjugated diene.

The urethanized (meth)acrylic compound (A-1) has a weight-average molecular weight of preferably 1,250 or more and less than 20,000, more preferably 1,500 or more and 17,500 or less, even more preferably 1,800 or more and 16,000 or less, particularly preferably 2,500 or more and 15,000 or less per polymerizable group. With the number of polymerizable groups confined in these ranges in the urethanized (meth)acrylic compound (A-1), crosslinkage takes place in a moderate fashion, and it is possible to more effectively maintain the mechanical strength while reducing polymerization shrinkage stress.

· (Meth)Acrylic Compound (A-2) Having no Urethane Skeleton

The (meth)acrylic compound (A-2) having no urethane skeleton has a structure (a polymer skeleton) selected from the group consisting of a polyester, a polycarbonate, a polyurethane, a polyether, a poly-conjugated diene, and a hydrogenated poly-conjugated diene. These are not particularly limited, as long as the above structure is present.

Examples of the polyester include a polymer of phthalic acid and an alkylene diol having 2 to 12 carbon atoms, a polymer of adipic acid and an alkylene glycol having 2 to 12 carbon atoms, a polymer of maleic acid and an alkylene diol having 2 to 12 carbon atoms, a polymer of β-propiolactone, a polymer of γ-butyrolactone, a polymer of 6-valerolactone, a polymer of ε-caprolactone, and a copolymer of these.

Examples of the polycarbonate include a polycarbonate derived from an aliphatic diol having 2 to 12 carbon atoms, a polycarbonate derived from bisphenol A, and a polycarbonate derived from a C2 to C12 aliphatic diol and bisphenol A.

Examples of the polyurethane include a polymer of a C2 to C12 aliphatic diol and a C1 to C12 diisocyanate.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these.

In view of superior flexibility and superior water resistance, preferred among these are the polyester, polycarbonate, and poly-conjugated diene structures. A polyol having the polymer skeleton mentioned above can be used for the production of (meth)acrylic compound (A-2) having no urethane skeleton. The glass transition temperature and acetone solubility of the (meth)acrylic compound (A-2) having no urethane skeleton can be adjusted by adjusting the skeleton or molecular weight of a structure (polymer skeleton) selected from the group consisting of a polyester, a polycarbonate, a polyurethane, a polyether, a poly-conjugated diene, and a hydrogenated poly-conjugated diene.

Examples of the (meth)acrylic compound having a hydroxyl group include:

hydroxy (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis[4-[3-(meth)acryloyloxy-2-hydroxypropoxy]phenyl]propane, 1,2-bis[3-(meth)acryloyloxy-2-hydroxypropoxy]ethane, pentaerythritol tri(meth)acrylate, and tri or tetra(meth)acrylates of dipentaerythritol; and

hydroxy (meth)acrylamide compounds such as N-hydroxyethyl (meth)acrylamide, and N,N-bis(2-hydroxyethyl)(meth)acrylamide.

The (meth)acrylic compound (A-2) having no urethane skeleton obtained is, for example, a product of a reaction of any combination of: the polyol having at least one structure selected from the group consisting of a polyester, a polycarbonate, a polyurethane, a polyether, a poly-conjugated diene, and a hydrogenated poly-conjugated diene; and the (meth)acrylic compound having a hydroxyl group.

The crosslink density increases, and it may not be possible to sufficiently reduce polymerization shrinkage

stress when the (meth)acrylic compound (A-2) having no urethane skeleton has an excessively large number of polymerizable groups. On the other hand, the crosslink density decreases, and the mechanical strength may decrease when the number of polymerizable groups is too small. In this respect, the (meth)acrylic compound (A-2) having no urethane skeleton has a weight-average molecular weight of preferably 1,250 or more and less than 20,000, more preferably 1,500 or more and 17,500 or less, even more preferably 1,800 or more and 16,000 or less, particularly preferably 2,500 or more and 15,000 or less per polymerizable group.

· Compound Having Weight-Average Molecular Weight of 1,000 or More with no Polymerizable Group

The compound having a weight-average molecular weight of 1,000 or more with no polymerizable group can be classified into a compound (A-3) having a weight-average molecular weight of 1,000 or more with a urethane skeleton and with no polymerizable group (hereinafter, also referred to as "compound (A-3)"), and a compound (A-4) having a weight-average molecular weight of 1,000 or more with no urethane skeleton or polymerizable group (hereinafter, also referred to as "compound (A-4)"). Preferred is compound (A-3). A certain embodiment is, for example, a dental composition that comprises a compound (A), a monomer (B), and a polymerization initiator (C), but does not comprise a compound (A-3) and/or a compound (A-4).

The compound (A-3) has a structure (a polymer skeleton) selected from the group consisting of a polyester, a polycarbonate, a polyurethane, a polyether, a poly-conjugated diene, and a hydrogenated poly-conjugated diene, in addition to a urethane bond. These are not particularly limited, as long as the above structure is present.

Examples of the polyester include a polymer of phthalic acid and an alkylene diol having 2 to 12 carbon atoms, a polymer of adipic acid and an alkylene glycol having 2 to 12 carbon atoms, a polymer of maleic acid and an alkylene diol having 2 to 12 carbon atoms, a polymer of β-propiolactone, a polymer of γ-butyrolactone, a polymer of 6-valerolactone, a polymer of ε-caprolactone, and a copolymer of these.

Examples of the polycarbonate include a polycarbonate derived from an aliphatic diol having 2 to 12 carbon atoms, a polycarbonate derived from bisphenol A, and a polycarbonate derived from a C2 to C12 aliphatic diol and bisphenol A.

Examples of the polyurethane include a polymer of a C2 to C12 aliphatic diol and a C1 to C12 diisocyanate.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these.

In view of superior flexibility and superior water resistance, preferred among these are the polyester, polycarbonate, and poly-conjugated diene structures. A polyol having the polymer skeleton mentioned above can be used for the production of the compound having a weight-average molecular weight of 1,000 or more with no polymerizable group. The glass transition temperature and acetone solubility of the compound (A-3) can be adjusted by adjusting the skeleton or molecular weight of a structure (polymer skeleton) selected from the group consisting of a polyester, a polycarbonate, a polyurethane, a polyether, a poly-conjugated diene, and a hydrogenated poly-conjugated diene.

Examples of the compound having an isocyanate group include hexamethylene diisocyanate (HDI), tolylene diisocyanate (TDI), xylylene diisocyanate (XDI), diphenylmethane diisocyanate (MDI), isophorone diisocyanate (IPDI), trimethylhexamethylene diisocyanate (TMHMDI), tricyclodecane diisocyanate (TCDDI), and adamantane diisocyanate (ADI).

The compound having a hydroxyl group is not particularly limited, and known compounds can be used, as long as it does not have a polymerizable group. The compound having a hydroxyl group may be, for example, the polyol described above.

The addition reaction of the compound having a isocyanate group and the compound having a hydroxyl group can be carried out following a known method, and is not particularly limited.

The compound (A-3) obtained may be, for example, the polyol having at least one structure selected from the group consisting of a polyester, a polycarbonate, a polyurethane, a polyether, a poly-conjugated diene, and a hydrogenated poly-conjugated diene; or a product of a reaction between the polyol and a compound having an isocyanate group.

Because the weight-average molecular weight (Mw) of compound (A) is a contributing factor for providing the desired solubility in acetone, and in view of viscosity and the polymerization shrinkage stress reducing effect, the compound (A) has a weight-average molecular weight (Mw) of 1,000 to 80,000, preferably 2,000 to 50,000, more preferably 3,000 to 20,000. In the present invention, a weight-average molecular weight (Mw) means a weight-average molecular weight determined by gel permeation chromatography (GPC) in terms of polystyrene, and can be measured by using a known method.

The polarity difference between compound (A) and monomer (B) is important for obtaining the polymerization shrinkage stress reducing effect, and, to this end, the compound (A) must have a lower solubility in acetone relative to

the monomer (B) having a higher solubility in acetone. In view of the compatibility with the monomer component (B), the compound (A) must have a solubility in acetone of less than 50 g/L, preferably less than 30 g/L, more preferably less than 25 g/L, even more preferably less than 20 g/L, particularly preferably less than 10 g/L.

A solubility parameter (SP) value may be used as a parameter that measures the difference in polarity and compatibility between compound (A) and monomer (B). The SP value is a measure of solubility. Higher SP values indicate higher polarity, and lower SP values indicate lower polarity. In view of the polymerization shrinkage stress reducing effect, the compound (A) and monomer (B) have an SP value difference of preferably 1.0 $(cal/cm^3)^{1/2}$ or more, more preferably 2.0 $(cal/cm^3)^{1/2}$ or more, even more preferably 3.0 $(cal/cm^3)^{1/2}$ or more, particularly preferably 4.0 $(cal/cm^3)^{1/2}$ or more. The SP value can be measured as follows. A sample is weighed into a 100 mL Erlenmeyer flask in the amount of 0.5 g, and 10 mL of acetone is added to dissolve the sample. Hexane is dropped into the mixture while stirring the mixture with a magnetic stirrer, and the volume of hexane (vh) that produced cloudiness in the solution (cloud point) is determined. Subsequently, the volume of deionized water (vd) at the cloud point is determined by adding deionized water instead of hexane. The SP value can then be determined from vh and vd using the formula presented in reference literature SUH, CLARKE, J. P. S. A-1, 5, 1671 to 1681 (1967). When this method cannot be used to find the SP value such as when the sample does not dissolve in acetone, the SP value can be estimated using the method proposed by Fedors et al. Specifically, the SP value can be determined by referring to Polymer Engineering and Science, February, 1974, Vol. 14, No. 2, ROBERF. FEDORS. (pp. 147 to 154). More specifically, the SP value can be calculated by the Fedors method using the following formula (A).

$$\text{SP value} = (\text{CED value})^{1/2} = (E/V)^{1/2} \qquad \text{Formula (A)}$$

In the formula (A), E is the cohesive energy (cal/mol), and V is the molar molecular volume ($cm^3$/mol).

The compound (A) is required to include at least one glass transition temperature (hereinafter, also referred to simply as "Tg") that is less than 40°C. Tg is not particularly limited, as long as it is less than 40°C. However, in view of providing a more superior polymerization shrinkage stress reducing effect, it is preferable that Tg fall in a temperature range of preferably -100°C to 30°C, more preferably -75°C to 15°C, even more preferably -60°C to 10°C. When the compound (A) solely has a Tg of 40°C or more, the compound (A) turns into a vitreous state at the temperature of polymerization, and fails to exhibit the polymerization shrinkage stress reducing effect. The compound (A) may have more than one Tg, as described below. In this case, one of the multiple glass transition temperatures Tg must be less than 40°C, while the other glass transition temperatures Tg may be in a temperature range of 40°C or more. For example, the compound (A) includes a compound having two glass transition temperatures Tg, one at -42°C, and the other at 44.6°C. In certain embodiments, the urethanized (meth)acrylic compound (A-1) is preferably a urethanized (meth)acrylic compound (A-1a) having no aromatic ring in its skeleton, in order to prevent an increase of glass transition temperature, and adjust the glass transition region to less than 40°C. In other embodiments, the urethanized (meth)acrylic compound (A-1) is preferably a urethanized (meth)acrylic compound (A-1b) that does not have a cyclic structure (an aromatic ring, a heterocyclic ring, or an alicyclic structure) in its skeleton, in order to adjust the glass transition region to less than 40°C.

Yet another embodiment is, for example, a dental composition that comprises a compound (A), a monomer (B) having a solubility of 50 g/L or more in acetone at 25°C, and a polymerization initiator (C), wherein the compound (A) has a weight-average molecular weight of 1,000 to 80,000, and includes a Tg of less than 40°C, in addition to having a solubility of less than 50 g/L in acetone at 25°C,

the dental composition comprising no compound that has a weight-average molecular weight of 2,000 or more, and that includes a Tg of less than 40°C, in addition to having a solubility of 50 g/L or more in acetone at 25°C (for example, such as a (meth)acrylic compound).

In the present invention, a glass transition temperature (Tg) means the midpoint glass transition temperature ($T_{mg}$). Specifically, a glass transition temperature (Tg) in the present invention can be determined by measurement based on JIS K 7121-1987 (rev. 2012). In the following, "baseline" means a DTA or DSC curve in a temperature range in which glass transitions or reactions do not occur in the measurement sample, as defined in JIS K 7121-1987 (rev. 2012). The midpoint glass transition temperature ($T_{mg}$) is the temperature where the straight line vertically equidistant from the straight line as an extension of each baseline intersects the curve where the glass transition shows a step-like change. FIG. 1 shows the extrapolated glass transition onset temperature ($T_{ig}$), extrapolated glass transition end temperature ($T_{eg}$), and midpoint glass transition temperature ($T_{mg}$). The extrapolated glass transition onset temperature ($T_{ig}$) is the temperature where a straight line as an extension of the baseline on the low-temperature side toward the high-temperature side intersects the tangent to the steepest slope of the curve where the glass transition shows a step-like change. The extrapolated glass transition end temperature ($T_{eg}$) is the temperature where the straight line as an extension of the baseline on the high-temperature side toward the low-temperature side intersects the tangent to the steepest slope of the curve where the glass transition shows a step-like change. In heat flux DSC, the extrapolated glass transition end temperature ($T_{eg}$) of when a peak appears on the high-temperature side of a step-like change is the

temperature where the straight line as an extension of the baseline on the high-temperature side toward the low-temperature side intersects the tangent to the steepest slope of the curve on the high-temperature side of the peak.

The measurement method and measurement conditions related to the glass transition temperature of the present invention are as described in the EXAMPLES section below using heat flux DSC.

In certain embodiments, the compound (A) preferably has two or more glass transition temperatures, more preferably two glass transition temperatures. In two or more glass transition temperatures Tg, it is preferable that one or more glass transition temperatures Tg be present on the low-temperature side and on the high-temperature side. In such embodiments, a first glass transition temperature Tg (on the low-temperature side) is preferably -100°C or more and 20°C or less, more preferably -75°C or more and 15°C or less, even more preferably -60°C or more and 10°C or less. In such embodiments, a second glass transition temperature Tg (on the high-temperature side) is preferably 20°C or more and less than 80°C, more preferably 25°C or more and less than 70°C, even more preferably 30°C or more and less than

65°C. A certain embodiment is, for example, a dental composition in which the compound (A) has one or more glass transition temperatures in the temperature region of -100°C or more and 20°C or less, and one or more glass transition temperatures in the temperature region of 20°C or more and less than 80°C.

Another certain embodiment is, for example, a dental composition that comprises a compound (A), a monomer (B) having a solubility of 50 g/L or more in acetone at 25°C, and a polymerization initiator (C), wherein the compound (A) has a weight-average molecular weight of 1,000 to 80,000, and includes a Tg of less than 40°C, in addition to having a solubility of less than 50 g/L in acetone at 25°C,
the dental composition comprising no compound that has a weight-average molecular weight of 2,000 or more, and that does not have two or more glass transition temperatures (for example, such as a (meth)acrylic compound).

Yet another embodiment is, for example, a dental composition that comprises a compound (A), a monomer (B) having a solubility of 50 g/L or more in acetone at 25°C, and a polymerization initiator (C), wherein the compound (A) has a weight-average molecular weight of 1,000 to 80,000, and includes a Tg of less than 40°C, in addition to having a solubility of less than 50 g/L in acetone at 25°C,
the dental composition comprising no (meth)acrylic compound that has a weight-average molecular weight of 5,000 to 50,000, and a weight-average molecular weight of 1,250 or more and less than 20,000 per (meth)acryl group, and that does not have two or more glass transition temperatures.

In view of ease of handling and the polymerization shrinkage stress reducing effect, the compound (A) has a viscosity at 25°C of preferably 1,000 to 10,000,000 cps, more preferably 5,000 to 7,500,000 cps, even more preferably 10,000 to 7,000,000 cps. In the present invention, viscosity means a viscosity measured at 25°C with a Brookfield viscometer. Measurement conditions, such as time and rotational speed, are appropriately adjusted according to the viscosity range.

The compound (A) may be a commercially available product. Examples of such commercially available products include urethane polymers having a terminal polymerizable group, such as the Art Resin series (UN-7600, UN7700) manufactured by Negami Chemical Industrial Co., Ltd.; the Kuraprene series (polyisoprene skeleton or polybutadiene skeleton) manufactured by Kuraray Co., Ltd. (LIR-30, LIR-50, LIR-390, LIR-403, LIR-410, UC-102M, UC-203M, LIR-700, LBR-302, LBR-307, LBR-305, LBR-352, LBR-361, L-SBR-820, L-SBR-841); polyols manufactured by Kuraray Co., Ltd. (P-6010, P-5010, P-4010, P-3010, P-2010, P-1010, F-3010, F2010, F-1010, P-2011, P-1020, P-2020, P-530, P-2030, P-2050, C-2090); and the liquid polybutadiene NISSO-PB manufactured by Nippon Soda Co., Ltd. (B-1000, B-2000, B-3000, BI-2000, BI-3000, G-1000, G-2000, G-3000, GI-1000, GI-2000, GI-3000, TEAI-1000, TE-2000, TE-4000, JP-100, JP-200). Preferred are UN-7600, UN7700, LBR-302, LBR-307, LBR-305, LBR-352, LBR-361, L-SBR-820, UC-102M, UC-203M, C-2090, P-2020, P-2050, B3000, BI-2000, BI-3000, TEAI-1000, TE-2000, and TE-4000. More preferred are UN-7600, UC-102M, TE-2000, and TE-4000.

In view of the mechanical strength, paste properties, and polymerization shrinkage stress reducing effect, the content of the compound (A) in a dental composition of the present invention is preferably 0.1 to 50 parts by mass with respect to total 100 parts by mass of compound (A) and monomer (B). In view of mechanical strength and ease of undergoing a phase separation from the monomer (B) to form a distinct phase such as by forming an island-in-sea structure, the content of compound (A) is more preferably 0.5 to 40 parts by mass, even more preferably 1 to 35 parts by mass, yet more preferably 1 to 25 parts by mass, particularly preferably 1 to 18 parts by mass. In view of the mechanical strength, paste properties, and polymerization shrinkage stress reducing effect, it is preferable in certain embodiments that the content of compound (A) be 0.1 mass% or more, more preferably 0.5 mass% or more, even more preferably 1 mass% or more in a total amount of the dental composition. In view of the mechanical strength, paste properties, and polymerization shrinkage stress reducing effect, the content of compound (A) is preferably 30 mass% or less, more preferably 25 mass% or less, even more preferably 20 mass% or less in a total amount of the dental composition.

[Monomer (B)]

The monomer (B) used has a high solubility in acetone, specifically a solubility of 50 g/L or more, preferably 75 g/L or more, more preferably 90 g/L or more in acetone at 25°C. The monomer (B) may be used alone, or two or more thereof may be used in combination. The monomer (B) may be, for example, a monomer (B-1) having an acidic group, a hydrophobic monomer (B-2) having no acidic group, or a hydrophilic monomer (B-3) having no acidic group. When using two or more monomers (B), it is possible to use a mixture of monomers (B) having a high solubility in acetone (a mixture with a solubility of 50 g/L or more in acetone at 25°C).

Compound (A) and monomer (B) become less compatible with each other as the difference in polarity between compound (A) and monomer (B) increases, and more easily segregate into two phases of monomer (B) component and compound (A) component such as by forming an island-in-sea structure. It is accordingly preferable that the difference in solubility in acetone between monomer (B) and compound (A) (solubility in acetone of monomer (B) - solubility in acetone of compound (A)) be 10 g/L or more, more preferably 20 g/L or more, even more preferably 30 g/L or more. The difference in solubility in acetone between monomer (B) and compound (A) does not have specific upper limits because a phase separation more easily occurs as the polarity difference increases. However, the solubility difference may be 300 g/L or less, 200 g/L or less, or 150 g/L or less. In such embodiments, the low glass transition temperature of compound (A) allows it to more easily form an even more flexible phase, and provides a dental composition having an even more superior polymerization shrinkage stress reducing effect.

· Monomer (B-1) Having Acidic Group

The monomer (B-1) having an acidic group has an acid etching effect and a priming effect, and is a component that provides demineralizing effect and penetrative effect. The monomer (B-1) having an acidic group is polymerizable, and provides curing effect. The adhesive properties to tooth structure, and bond durability improve by containing the monomer (B-1) having an acidic group.

The monomer (B-1) having an acidic group is, for example, a monomer having at least one acidic group (such as a phosphoric acid group, a pyrophosphoric acid group, a thiophosphoric acid group, a phosphonic acid group, a sulfonic acid group, and a carboxylic acid group), and at least one polymerizable group (such as a (meth)acryloyl group, a vinyl group, and a styrene group). In view of adhesive properties to tooth structure, the monomer (B-1) having an acidic group is preferably a phosphoric acid group-containing monomer. Specific examples of the monomer (B-1) having an acidic group are as follows.

Examples of the phosphoric acid group-containing monomer include 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl dihydrogen phosphate, 2-(meth)acryloyloxyethylphenyl hydrogen phosphate, 2-(meth)acryloyloxyethyl-2-bromoethyl hydrogen phosphate, bis[2-(meth)acryloyloxy-(1-hydroxymethyl)ethyl]hydrogen phosphate, and acid chlorides, alkali metal salts, and ammonium salts of these.

Examples of the pyrophosphoric acid group-containing monomer include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides, alkali metal salts, and ammonium salts of these.

Examples of the thiophosphoric acid group-containing monomer include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, 20-(meth)acryloyloxyicosyl dihydrogen thiophosphate, and acid chlorides, alkali metal salts, and ammonium salts of these.

Examples of the phosphonic acid group-containing monomer include 2-(meth)acryloyloxyethylphenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl-3-phosphonoacetate, 10-(meth)acryloyloxydecyl-3-phosphonoacetate, and acid chlorides, alkali metal salts, and ammonium salts of these.

Examples of the sulfonic acid group-containing monomer include 2-(meth)acrylamide-2-methylpropanesulfonic acid, styrenesulfonic acid, and 2-sulfoethyl (meth)acrylate.

Examples of the carboxylic acid group-containing monomer include monomers having one carboxy group within the molecule, and monomers having a plurality of carboxy groups within the molecule.

Examples of monomers having one carboxy group within the molecule include (meth)acrylic acid, N-(meth)acryloylglycine, N-(meth)acryloylaspartic acid, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, p-vinyl benzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, 2-(meth)acryloyloxyethyl hydrogen succinate, 2-(meth)acryloyloxyethyl hydrogen phthalate, 2-(meth)acryloyloxyethyl hydrogen malate, and acid halides of these.

Examples of monomers having a plurality of carboxy groups within the molecule include 6-(meth)acryloyloxyhexane-1,1-dicarboxylic acid, 9- (meth)acryloyloxynonane-1,1 -dicarboxylic acid, 10-(meth)acryloyloxydecane-1,1-dicarboxylic acid, 11-(meth)acryloyloxyundecane-1,1-dicarboxylic acid, 12-(meth)acryloyloxydodecane-1,1-dicarboxylic acid, 13-(meth)acryloyloxytridecane-1,1-dicarboxylic acid, 4-(meth)acryloyloxyethyltrimellitate, 4-(meth)acryloyloxyethyltrimellitate anhydride, 4-(meth)acryloyloxybutyltrimellitate, 4-(meth)acryloyloxyhexyltrimellitate, 4-(meth)acryloyloxydecyltrimellitate, 2-(meth)acryloyloxyethyl-3'-(meth)acryloyloxy-2'-(3,4-dicarboxybenzoyloxy)propyl succinate, and acid anhydrides or acid halides of these.

Among these compounds, the monomer (B-1) having an acidic group is preferably a phosphoric acid group- or pyrophosphoric acid group-containing (meth)acrylic monomer, particularly a phosphoric acid group-containing (meth)acrylic monomer, because these monomers develop even more superior adhesive properties to tooth structure. In view of the ability to exhibit high adhesive properties by showing high demineralization in the absence of an organic solvent, the monomer (B-1) having an acidic group is even more preferably a divalent phosphoric acid group-containing (meth)acrylic monomer having a C6 to C20 alkyl or alkylene group as a backbone within the molecule, particularly preferably a divalent phosphoric acid group-containing (meth)acrylic monomer having a C8 to C12 alkylene group as a backbone within the molecule (e.g., 10-methacryloyloxydecyl dihydrogen phosphate).

The monomer (B-1) having an acidic group may be used alone, or two or more thereof may be used in combination. Excessively high or low contents of monomer (B-1) having an acidic group may result in a decrease of adhesive properties. In this respect, the content of monomer (B-1) having an acidic group is preferably 1 to 50 parts by mass, more preferably 3 to 40 parts by mass, even more preferably 5 to 30 parts by mass with respect to total 100 parts by mass of the compound (A) and the monomer (B) in the dental composition.

· Hydrophobic Monomer (B-2) Having no Acidic Group

The hydrophobic monomer (B-2) having no acidic group (hereinafter, referred to as "hydrophobic monomer (B-2)") improves the mechanical strength, ease of handling, and other properties of the dental composition. Preferably, the hydrophobic monomer (B-2) is a radical monomer having a polymerizable group, with no acidic group. In view of ease of radical polymerization, the polymerizable group is more preferably a (meth)acryl group, and/or a (meth)acrylamide group. A hydrophobic monomer (B-2) means a monomer that does not have an acidic group, and that has a solubility at 25°C of less than 10 mass% in water. Examples include crosslinkable monomers such as aromatic compound-based bifunctional monomers, aliphatic compound-based bifunctional monomers, and tri- and higher-functional monomers.

Examples of the aromatic compound-based bifunctional monomers include 2,2-bis((meth)acryloyloxyphenyl)propane, 2,2-bis[4-(3-(meth)acryloyloxy-2-hydroxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane. Preferred are 2,2-bis[4-(3-methacryloyloxy-2-hydroxypropoxy)phenyl]propane (commonly known as "Bis-GMA"), 2,2-bis(4-(meth)acryloyloxyethoxyphenyl)propane, 2,2-bis(4-methacryloyloxypolyethoxyphenyl)propane (average number of moles of ethoxy group added is 2.6; commonly known as "D-2.6E"), 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, and 2,2-bis(4-(meth)acryloyloxypentaethoxyphenyl)propane.

Examples of the aliphatic compound-based bifunctional monomers include glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1,2-bis(3-methacryloyloxy-2-hydroxypropoxy)ethane, 2,2,4-trimethylhexamethylenebis(2-carbamoyloxyethyl)di(meth)acrylate, N-methacryloyloxyethylacrylamide, and N-methacryloyloxypropylamide. Preferred are triethylene glycol diacrylate, triethylene glycol dimethacrylate (commonly known as "3G"), neopentyl glycol di(meth)acrylate, 1,2-bis(3-methacryloyloxy-2-hydroxypropoxy)ethane, 2,2,4-trimethylhexamethylenebis(2-carbamoyloxyethyl)dimethacrylate (commonly known as "UDMA"), 1,10-decanediol dimethacrylate (commonly known as "DD"), 2,2,4-trimethylhexamethylenebis(2-carbamoyloxyethyl)dimethacrylate, and N-methacryloyloxyethylacrylamide (commonly known as "MAEA").

Examples of the tri- and higher-functional monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acr ylate, and 1,7-diacryloyloxy-2,2,6,6-tetra(meth)acryloyloxymethyl-4-oxaheptane. Preferred is N,N-(2,2,4-trimethylhexamethylene)bis[2-(aminocarboxy)propane-1,3-diol]tetramethacryl ate.

In view of mechanical strength and ease of handling, preferred for use as hydrophobic monomer (B-2) are aromatic compound-based bifunctional monomers and aliphatic compound-based bifunctional monomers. Preferred as aromatic compound-based bifunctional monomers are Bis-GMA and D-2.6E. Preferred as aliphatic compound-based bifunctional monomers are glycerol di(meth)acrylate, 3G, neopentyl glycol di(meth)acrylate, 1,6-hexanediol di(meth)acrylate, DD, 1,2-bis(3-methacryloyloxy-2-hydroxypropoxy)ethane, UDMA, and MAEA.

In view of initial adhesion to tooth structure, bond durability, and mechanical strength, the hydrophobic monomer (B-2) is more preferably Bis-GMA, D-2.6E, 3G, UDMA, DD, or MAEA, even more preferably D-2.6E, DD, or MAEA.

The hydrophobic monomer (B-2) may be contained alone, or two or more thereof may be used in combination. An excessively high content of hydrophobic monomer (B-2) may result in a decrease of adhesion due to a decrease of penetrability into the tooth structure, whereas the effect to improve mechanical strength may become insufficient with excessively low contents of hydrophobic monomer (B-2). In this respect, the content of hydrophobic monomer (B-2) is preferably 20 to 99 parts by mass, more preferably 40 to 95 parts by mass, even more preferably 60 to 92 parts by mass with respect to total 100 parts by mass of the compound (A) and the monomer (B) in the dental composition. In certain embodiments, in view of the mechanical strength, paste properties, and polymerization shrinkage stress reducing effect, the content of hydrophobic monomer (B-2) is preferably 1 mass% or more, more preferably 5 mass% or more, even more preferably 10 mass% or more in a total amount of the dental composition. In view of the mechanical strength, paste properties, and polymerization shrinkage stress reducing effect, the content of the hydrophobic monomer (B-2) is preferably 48 mass% or less, more preferably 40 mass% or less, even more preferably 35 mass% or less in a total amount of the dental composition.

· Hydrophilic Monomer (B-3) Having no Acidic Group

Preferably, a dental composition of the present invention additionally comprises a hydrophilic monomer (B-3) having no acidic group (hereinafter, referred to as "hydrophilic monomer (B-3)"). The hydrophilic monomer (B-3) promotes penetration of the components of the dental composition into tooth structure. The hydrophilic monomer (B-3) itself also penetrates into tooth structure, and adheres to the organic component (collagen) in the tooth structure. The hydrophilic monomer (B-3) is preferably a radical monomer having a polymerizable group, with no acidic group. In view of ease of radical polymerization, the polymerizable group is more preferably a (meth)acryl group and/or a (meth)acrylamide group. A hydrophilic monomer (B-3) means a monomer that does not have an acidic group, and that has a solubility at 25°C of 10 mass% or more in water. Preferably, the hydrophilic monomer (B-3) is one having a solubility at 25°C of 30 mass% or more, more preferably one that can dissolve in water in any fractions at 25°C. The hydrophilic monomer (B-3) is preferably one having a hydrophilic group such as a hydroxyl group, an oxymethylene group, an oxyethylene group, an oxypropylene group, or an amide group. Examples of such monomers include hydrophilic monofunctional (meth)acrylate monomers such as 2-hydroxyethyl (meth)acrylate (HEMA), 3-hydroxypropyl (meth)acrylate, 2-hydroxypropyl (meth)acrylate, 1,3-dihydroxypropyl (meth)acrylate, 2,3-dihydroxypropyl (meth)acrylate, 2-((meth)acryloyloxy)ethyltrimethylammonium chloride, and polyethylene glycol di(meth)acrylate (with nine or more oxyethylene groups); and hydrophilic monofunctional (meth)acrylamide monomers such as N-methylol(meth)acrylamide, N-hydroxyethyl(meth)acrylamide, N,N-bis(2-hydroxyethyl)(meth)acrylamide, N-methoxymethyl(meth)acrylamide, N-ethoxymethyl(meth)acrylamide, diacetone(meth)acrylamide, 4-(meth)acryloylmorpholine, N-trihydroxymethyl-N-methyl(meth)acrylamide, N,N-dimethylacrylamide, and N,N-diethylacrylamide.

In view of adhesive properties to tooth structure, preferred as hydrophilic monomer (B-3) are 2-hydroxyethyl (meth)acrylate, 2,3-dihydroxypropyl (meth)acrylate, and hydrophilic monofunctional (meth)acrylamide monomers. More preferred are 2-hydroxyethyl (meth)acrylate, N,N-dimethylacrylamide, and N,N-diethylacrylamide. The hydrophilic monomer (B-3) may be contained alone, or two or more thereof may be used in combination.

It may not be possible to obtain a sufficient adhesion improving effect with an excessively low content of hydrophilic monomer (B-3) in the present invention, whereas the mechanical strength may decrease when the content of hydrophilic monomer (B-3) is excessively high. It is therefore preferable that the content of hydrophilic monomer (B-3) be 0 to 50 parts by mass, more preferably 0 to 40 parts by mass, even more preferably 0 to 30 parts by mass with respect to total 100 parts by mass of the compound (A) and the monomer (B) in the dental composition. The content of hydrophilic monomer (B-3) may be 0 part by mass.

The difference in solubility in acetone at 25°C between compound (A) and monomer (B) is preferably 30 g/L or more, more preferably 40 g/L or more, even more preferably 50 g/L or more because a more distinct phase separation can occur in a dental composition of the present invention.

[Polymerization Initiator (C)]

The polymerization initiator (C) can be broadly classified into photopolymerization initiator and chemical polymerization initiator, with the photopolymerization initiator further divided into water-soluble photopolymerization initiator (C-1) and water-insoluble photopolymerization initiator (C-2). The polymerization initiator (C) may be solely a water-soluble photopolymerization initiator (C-1) or a water-insoluble photopolymerization initiator (C-2), or a combination of water-soluble photopolymerization initiator (C-1) and water-insoluble photopolymerization initiator (C-2).

· Water-Soluble Photopolymerization Initiator (C-1)

The water-soluble photopolymerization initiator (C-1) can achieve high bond strength by improving the polymerization curability at the hydrophilic tooth interface. The water-soluble photopolymerization initiator (C-1) has a solubility in 25°C water of 1.0 mass% or more, preferably 1.5 mass% or more, more preferably 2.0 mass% or more, even more preferably 2.5 mass% or more. When the solubility is less than 1.0 mass%, the water-soluble photopolymerization initiator (C-1) does not sufficiently dissolve in water in tooth structure, and fails to sufficiently promote polymerization at the bond interface.

Examples of the water-soluble photopolymerization initiator (C-1) include water-soluble acylphosphine oxides, water-soluble thioxanthones, and $\alpha$-hydroxyalkylacetophenones. The $\alpha$-hydroxyalkylacetophenones may be, for example, compounds having a (poly)ethylene glycol chain introduced into the hydroxyl group(s) of 1-[4-(2-hydroxyethoxy)-phenyl]-2-hydroxy-2-methyl-1-propan-1-one, compounds having a (poly)ethylene glycol chain introduced into the hydroxyl group and/or phenyl group of 1-hydroxycyclohexyl phenyl ketone, compounds having $-OCH_2COO^-Na^+$ introduced into the phenyl group of 1-hydroxycyclohexyl phenyl ketone, compounds having a (poly)ethylene glycol chain introduced into the hydroxyl group and/or phenyl group of 2-hydroxy-2-methyl-1-phenylpropan-1-one, and compounds having $-OCH_2COO^-Na^+$ introduced into the phenyl group of 2-hydroxy-2-methyl-1-phenylpropan-1-one. Other examples of the water-soluble photopolymerization initiator (C-1) include quaternary ammonium salt compounds prepared by quaternization of the amino group of $\alpha$-aminoalkylphenones, such as 2-methyl-1[4-(methylthio)phenyl]-2-morpholinopropan-1-one, and 2-benzyl-2-dimethylamino-1-(4-morpholinophenyl)butanone-1.

The water-soluble thioxanthones may be any of, for example,

2-hydroxy-3-(9-oxo-9H-thioxanthen-4-yloxy)-N,N,N-trimethyl-1-propaneaminium chloride,
2-hydroxy-3-(1-methyl-9-oxo-9H-thioxanthen-4-yloxy)-N,N,N-trimethyl-1-propaneaminiu m chloride,
2-hydroxy-3-(9-oxo-9H-thioxanthen-2-yloxy)-N,N,N-trimethyl-1-propaneaminium chloride,
2-hydroxy-3-(3,4-dimethyl-9-oxo-9H-thioxanthen-2-yloxy)-N,N,N-trimethyl-1-propaneami nium chloride,
2-hydroxy-3-(3,4-dimethyl-9H-thioxanthen-2-yloxy)-N,N,N-trimethyl-1-propaneaminium chloride, and
2-hydroxy-3-(1,3,4-trimethyl-9-oxo-9H-thioxanthen-2-yloxy)-N,N,N-trimethyl-1-propanea minium chloride.

The water-soluble acylphosphine oxides may be, for example, acylphosphine oxides represented by the following general formula (1) or (2).

[Chem. 1]

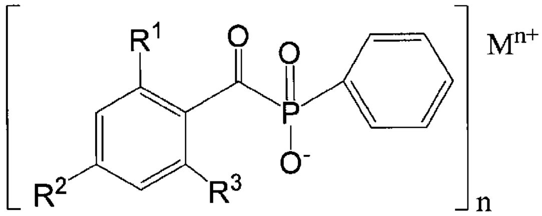

(1)

[Chem. 2]

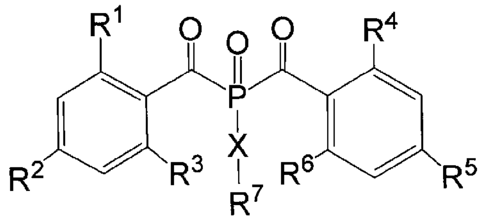

(2)

In the formulae, $R^1$, $R^2$, $R^3$, $R^4$, $R^5$, and $R^6$ are each independently a C1 to C4 linear or branched alkyl group or a halogen atom, M is a hydrogen ion, an alkali metal ion, an alkali earth metal ion, a magnesium ion, a pyridinium ion (the pyridine ring may have a substituent), or an ammonium ion represented by $HN^+R^8R^9R^{10}$ (where $R^8$, $R^9$, and $R^{10}$ are each independently an organic group or a hydrogen atom), n is 1 or 2, X is a C1 to C4 linear or branched alkylene group, $R^7$ represents $-CH(CH_3)COO(C_2H_4O)_pCH_3$, where p is an integer of 1 to 1,000.

The alkyl group represented by $R^1$, $R^2$, $R^3$, $R^4$, $R^5$, and $R^6$ is not particularly limited, as long as it is C1 to C4 linear or branched alkyl. Examples include methyl, ethyl, n-propyl, isopropyl, n-butyl, isobutyl, sec-butyl, 2-methylpropyl, and tert-butyl. The alkyl group represented by $R^1$, $R^2$, $R^3$, $R^4$, $R^5$, and $R^6$ is preferably a C1 to C3 linear alkyl group, more preferably methyl or ethyl, even more preferably methyl. Examples of the alkylene group represented by X include a methylene group, an ethylene group, an n-propylene group, an isopropylene group, and an n-butylene group. The alkylene group represented by X is preferably a C1 to C3 linear alkylene group, more preferably a methylene group or an ethylene group, even more preferably a methylene group.

Examples of the substituent of the pyridine ring when M is a pyridinium ion include halogen atoms (a fluorine atom, a chlorine atom, a bromine atom, an iodine atom), a carboxy group, a C2 to C6 linear or branched acyl group, a C1 to C6 linear or branched alkyl group, and a C1 to C6 linear or branched alkoxy group. Preferably, M is an alkali metal ion, an alkali earth metal ion, a magnesium ion, a pyridinium ion (the pyridine ring may have a substituent), or an ammonium ion represented by $HN^+R^8R^9R^{10}$ (the symbols have the same meaning as above). Examples of the alkali metal ion include a lithium ion, a sodium ion, a potassium ion, a rubidium ion, and a cesium ion. Examples of the alkali earth metal ion include a calcium ion, a strontium ion, a barium ion, and a radium ion. The organic group represented by $R^8$, $R^9$, and $R^{10}$ may be the same group exemplified above for the substituent of the pyridine ring (excluding a halogen atom).

In view of storage stability and shade stability in the composition, particularly preferred are compounds in which $R^1$, $R^2$, $R^3$, $R^4$, $R^5$, and $R^6$ are all methyl groups. Examples of $M^{n+}$ include $Li^+$, $Na^+$, $K^+$, $Ca^{2+}$, $Mg^{2+}$, and ammonium ions derived from various amines. Examples of the amines include ammonia, trimethylamine, diethylamine, dimethylaniline, ethylenediamine, triethanolamine, N,N-dimethylaminomethacrylate, N,N-dimethylaminobenzoic acid and alkyl esters thereof, N,N-diethylaminobenzoic acid and alkyl esters thereof, and N,N-bis(2-hydroxyethyl)-p-toluidine. In view of adhesive properties, p in $R^7$ is preferably 1 or more, more preferably 2 or more, even more preferably 3 or more, particularly preferably 4 or more, and is preferably 1,000 or less, more preferably 100 or less, even more preferably 75 or less, particularly preferably 50 or less.

Particularly preferably, the water-soluble acylphosphine oxides are compounds represented by general formula (1) and in which M is Li, and compounds represented by general formula (2) and in which the moiety corresponding to the group represented by $R^7$ is synthesized from polyethylene glycol methylether methacrylate having a molecular weight of 950.

The water-soluble acylphosphine oxides having such structures can be synthesized according to known methods, and some are available as commercially available products. For example, the methods disclosed in JP S57-197289 A and WO2014/095724 can be used for the synthesis of the water-soluble acylphosphine oxides. The water-soluble photopolymerization initiator (C-1) may be used alone, or two or more thereof may be used in combination.

The water-soluble photopolymerization initiator (C-1) may be dissolved in the dental composition, or may be dispersed in the form of a powder in the composition, provided that the water-soluble photopolymerization initiator (C-1) can dissolve in water at the surface of the tooth structure (moist body), and can selectively increase the polymerization curability at the bond interface and inside the resin-impregnated layer.

When the water-soluble photopolymerization initiator (C-1) is dispersed in the composition in the form of a powder, the average particle diameter is preferably 500 μm or less, more preferably 100 μm or less, even more preferably 50 μm or less because the water-soluble photopolymerization initiator (C-1) is more likely to settle when the average particle diameter is excessively large. The average particle diameter is preferably 0.01 μm or more because the specific surface area of the powder overly increases, and the amount of powder that can be dispersed in the composition decreases when the average particle diameter is excessively small. Taken together, the average particle diameter of water-soluble photopolymerization initiator (C-1) preferably ranges from 0.01 to 500 μm, more preferably 0.01 to 100 μm, even more preferably 0.01 to 50 μm.

The average particle diameter of a powder of water-soluble photopolymerization initiator (C-1) can be determined by taking an electron micrograph of at least 100 particles, and calculating the volume average particle diameter from the captured image after an image analysis performed with image-analyzing particle size distribution measurement software (Mac-View, manufactured by Mountech Co., Ltd.).

The shape of the water-soluble photopolymerization initiator (C-1) when it is dispersed in the composition in the form of a powder is not particularly limited, and may be any of various shapes, including, for example, spherical, stylus, plate-like, and crushed shapes. The water-soluble photopolymerization initiator (C-1) can be prepared using a known method such as pulverization, freeze drying, or reprecipitation. In view of the average particle diameter of the powder obtained, freeze drying and reprecipitation are preferred, and freeze drying is more preferred.

In view of curability and other properties of the dental composition obtained, the content of water-soluble photopolymerization initiator (C-1) is preferably 0.01 to 20 parts by mass with respect to total 100 parts by mass of the compound (A) and the monomer (B) in the dental composition. In view of providing high initial adhesion and bond durability and reducing polymerization shrinkage stress, the content of water-soluble photopolymerization initiator (C-1) is more preferably 0.05 to 10 parts by mass, even more preferably 0.1 to 5 parts by mass. When the content of water-soluble photopolymerization initiator (C-1) is less than 0.01 parts by mass, polymerization may fail to sufficiently proceed at the bond interface, and the bond strength may decrease. When the content of water-soluble photopolymerization initiator (C-1) is more than 20 parts by mass, it may not be possible to obtain a sufficient bond strength when the water-soluble photopolymerization initiator (C-1) has low polymerization performance, in addition to making it difficult to sufficiently dissolve, disperse, or diffuse water-soluble photopolymerization initiator (C-1) in the dental composition.

· Water-Insoluble Photopolymerization Initiator (C-2)

In view of curability, a dental composition of the present invention may comprise a water-insoluble photopolymerization initiator (C-2) having a solubility in water of less than 1.0 mass% at 25°C (hereinafter, referred to as "water-insoluble photopolymerization initiator (C-2)"), other than the water-soluble photopolymerization initiator (C-1). The water-insoluble photopolymerization initiator (C-2) used in the present invention may be a known photopolymerization initiator. The water-insoluble photopolymerization initiator (C-2) may be contained alone, or two or more thereof may be contained in combination.

Examples of the water-insoluble photopolymerization initiator (C-2) include (bis)acylphosphine oxides, thioxanthones, ketals, α-diketones, coumarins, anthraquinones, benzoin alkyl ether compounds, and α-aminoketone compounds, excluding those exemplified for water-soluble photopolymerization initiator (C-1).

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, and benzoyl di(2,6-dimethylphenyl)phosphonate. Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,5,6-trimethylbenzoyl)-2,4,4-trimethylpentylphosphine oxide.

Examples of the thioxanthones include thioxanthone, and 2-chlorothioxanthen-9-one.

Examples of the ketals include benzyl dimethyl ketal, and benzyl diethyl ketal.

Examples of the α-diketones include diacetyl, benzyl, dl-camphorquinone, 2,3-pentadione, 2,3-octadione, 9,10-phenanthrenequinone, 4,4'-oxybenzyl, and acenaphthenequinone. Particularly preferred is dl-camphorquinone for its maximum absorption wavelength occurring in the visible light region.

Examples of the coumarin compounds include compounds mentioned in JP H9-3109 A and JP H10-245525 A, including, for example, 3,3'-carbonylbis(7-diethylaminocoumarin), 3-(4-methoxybenzoyl)coumarin, 3-thienoylcoumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazolyl)-7-(diethylamino)coumarin, 3-(2-benzothiazolyl)-7-(dibutylamino)coumarin, 3-(2-benzoimidazolyl)-7-(diethylamino)coumarin, 3-(2-benzothiazolyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1 -oxo-2-propenyl]-2,3,6,7-tetrahydro-1,1,7,7-tetramethy l-1H,5H,11H-[1]benzopyrrano[6,7,8-ij]quinolizin-11-one, and 10-(2-benzothiazolyl)-2,3,6,7-tetrahydro-1,1,7,7-tetramethyl-1H,5H,11H-[1]benzopyrrano [6,7,8-ij]quinolizin-11-one.

Particularly preferred among these coumarin compounds are 3,3'-carbonylbis(7-diethylaminocoumarin) and 3,3'-carbonylbis(7-dibutylaminocoumarin).

Examples of the anthraquinones include anthraquinone, 1-chloroanthraquinone, 2-chloroanthraquinone, 1-bromoanthraquinone, 1,2-benzanthraquinone, 1-methylanthraquinone, 2-ethylanthraquinone, and 1-hydroxyanthraquinone.

Examples of the benzoin alkyl ether compounds include benzoin methyl ether, benzoin ethyl ether, benzoin isopropyl ether, and benzoin isobutyl ether.

Examples of the $\alpha$-aminoketone compounds include 2-methyl-1-[4-(methylthio)phenyl]-2-morpholinopropan-1-one.

The water-insoluble photopolymerization initiator (C-2) is preferably at least one selected from the group consisting of a (bis)acylphosphine oxide, an $\alpha$-diketone, and a coumarin compound. In this way, a dental composition can be obtained that has desirable photocurability both in the visible light region and the near ultraviolet region, and that shows sufficient photocurability regardless of whether the light source used is a halogen lamp, a light emitting diode (LED), or a xenon lamp.

The content of water-insoluble photopolymerization initiator (C-2) is not particularly limited. However, in view of curability and other properties of the composition obtained, the content of water-insoluble photopolymerization initiator (C-2) preferably ranges from 0.01 to 10 parts by mass, more preferably 0.05 to 7 parts by mass, even more preferably 0.1 to 5 parts by mass with respect to total 100 parts by mass of the compound (A) and the monomer (B) in the dental composition. When the content of water-insoluble photopolymerization initiator (C-2) is more than 10 parts by mass, it may not be possible to obtain a sufficient bond strength when the polymerization initiator itself has low polymerization performance, in addition to raising a possibility of precipitation from the dental composition.

In the present invention, the mass ratio of water-soluble photopolymerization initiator (C-1) and water-insoluble photopolymerization initiator (C-2) [(C-1):(C-2)] is preferably 10:1 to 1:10, more preferably 7:1 to 1:7, even more preferably 5:1 to 1:5, particularly preferably 3:1 to 1:3. When the fraction of water-soluble photopolymerization initiator (C-1) in the mass ratio exceeds 10:1, the curability of the dental composition itself decreases, and the dental composition may have difficulty exhibiting high bond strength. When the fraction of water-insoluble photopolymerization initiator (C-2) in the mass ratio exceeds 1:10, the dental composition may have difficulty exhibiting high bond strength as a result of insufficient promotion of polymerization at the bond interface, though the curability of the dental composition itself increases.

[Chemical Polymerization Initiator]

A dental composition of the present invention may additionally comprise a chemical polymerization initiator, for which an organic peroxide is preferred. The organic peroxide used as the chemical polymerization initiator is not particularly limited, and known organic peroxides may be used. Typical examples of such organic peroxides include ketoneperoxides, hydroperoxides, diacyl peroxides, dialkyl peroxides, peroxyketals, peroxyesters, and peroxy dicarbonates. Specific examples of these organic peroxides include those mentioned in WO2008/087977.

[Filler (D)]

A dental composition of the present invention may further comprise a filler (D). In the present invention, the filler (D) can be broadly classified into organic fillers, inorganic fillers, and organic-inorganic composite fillers.

Examples of the materials of the organic fillers include polymethylmethacrylate, polyethylmethacrylate, a methyl methacrylate-ethyl methacrylate copolymer, crosslinked polymethylmethacrylate, crosslinked polyethylmethacrylate, polyamides, polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used as a mixture. The shape of the organic filler is not particularly limited, and the particle diameter of the filler may be appropriately selected for use.

Examples of the materials of the inorganic fillers include quartz, silica, alumina, silica-titania, silica-titania-barium oxide, silica-zirconia, silica-alumina, lanthanum glass, borosilicate glass, soda glass, barium glass, strontium glass, glass-ceramics, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, strontium calcium fluoroaluminosilicate glass, ytterbium oxide, and silica-coated ytterbium fluoride. These may be used alone, or two or more thereof may be used as a mixture. The shape of the inorganic filler is not particularly

limited, and the particle diameter of the filler may be appropriately selected for use. In view of considerations such as the ease of handling and mechanical strength of the composition obtained, the average particle diameter of the inorganic filler is preferably 0.001 to 50 μm, more preferably 0.001 to 10 μm, even more preferably 0.001 to 8 μm. In the present invention, when the inorganic filler is surface treated as below, the average particle diameter of the inorganic filler means the average particle diameter before surface treatment.

The inorganic filler may be, for example, an irregularly shaped filler or a spherical filler. In view of improving the mechanical strength of the composition, the inorganic filler used is preferably a spherical filler. Another advantage of using a spherical filler is that the dental composition of the present invention, when used as a self-adhesive dental composite resin, can produce a composite resin having excellent surface gloss. Here, the spherical filler used in the present invention is a filler having an average uniformity of 0.6 or more as measured for round-shaped particles observed in a unit field of an electron micrograph of filler by dividing the diameter of a particle perpendicular to its maximum diameter by the maximum diameter. The spherical filler has an average particle diameter of preferably 0.05 to 5 μm. When the average particle diameter is less than 0.05 μm, the mechanical strength may decrease as a result of a decrease in the filling rate of the spherical filler in the composition. When the average particle diameter is more than 5 μm, the spherical filler has a reduced surface area, and the dental composition may fail to produce a cured product having high mechanical strength.

In order to adjust the flowability of the dental composition, the inorganic filler may be used after an optional surface treatment with a known surface treatment agent such as a silane coupling agent. Examples of the surface treatment agent include vinyltrimethoxysilane, vinyltriethoxysilane, vinyltrichlorosilane, vinyl tri(β-methoxyethoxy)silane, γ-methacryloyloxypropyltrimethoxysilane, 8-methacryloyloxyoctyltrimethoxysilane, 11-methacryloyloxyundecyltrimethoxysilane, γ-glycidoxypropyltrimethoxysilane, γ-mercaptopropyltrimethoxysilane, and γ-aminopropyltriethoxysilane.

The organic-inorganic composite filler used in the present invention is a filler obtained by adding a monomer compound to the inorganic filler, polymerizing the mixture in paste form, and pulverizing the polymerized filler. The organic-inorganic composite filler may be, for example, a TMPT filler (a filler obtained by mixing trimethylolpropane methacrylate and a silica filler, and pulverizing the mixture after polymerization). The shape of the organic-inorganic composite filler is not particularly limited, and may be determined by appropriately selecting the particle size of the filler. In view of properties such as the ease of handling and mechanical strength of the composition obtained, the organic-inorganic composite filler has an average particle diameter of preferably 0.001 to 50 μm, more preferably 0.001 to 10 μm.

In this specification, the average particle diameter of filler can be determined using a laser diffraction scattering method or by observing particles with an electron microscope. Specifically, a laser diffraction scattering method is more convenient for the measurement of particles of 0.1 μm or more, whereas electron microscopy is a more convenient method of particle size measurement for ultrafine particles of less than 0.1 μm. Here, 0.1 μm is a measured value by a laser diffraction scattering method.

As a specific example of a laser diffraction scattering method, the particle size may be measured by volume using, for example, a laser diffraction particle size distribution analyzer (SALD-2300, manufactured by Shimadzu Corporation) with a 0.2% sodium hexametaphosphate aqueous solution used as dispersion medium.

In electron microscopy, for example, particles may be photographed with an electron microscope (Model S-4000, manufactured by Hitachi), and the size of particles (at least 200 particles) observed in a unit field of the captured image may be measured using image-analyzing particle-size-distribution measurement software (Mac-View; Mountech Co., Ltd.). Here, the particle diameter is determined as an arithmetic mean value of the maximum and minimum lengths of particles, and the average primary particle diameter is calculated from the number of particles and the particle diameter.

The filler (D) used in the present invention may be a mixture or combination of two or more kinds of fillers of different materials having different particle size distributions and different forms. Unintended inclusion of non-filler particles as impurities is acceptable to such an extent that it is not detrimental to the effects of the present invention.

The content of the filler (D) used in the present invention is not particularly limited. Preferably, the dental composition comprises 0 to 2,000 parts by mass of filler (D) with respect to total 100 parts by mass of the compound (A) and the monomer (B) in the dental composition. In certain embodiments, in view of the present invention producing more superior effects such as mechanical strength, the content of filler (D) is preferably 50 mass% or more, more preferably 55 mass%, even more preferably 60 mass% in a total amount of the dental composition. In view of the present invention producing more superior effects such as mechanical strength, the content of filler (D) is preferably 98 mass% or less, more preferably 94 mass% or less, even more preferably 88 mass% or less in a total amount of the dental composition. Because the preferred content of filler (D) greatly differs in different embodiments, the preferred content of filler (D) suited for each embodiment is presented below along with descriptions of specific embodiments of a dental composition of the present invention.

[Polymerization Accelerator (E)]

In certain embodiments, a polymerization accelerator (E) is used with the water-insoluble photopolymerization

initiator (C-2) and/or the chemical polymerization initiator. Examples of the polymerization accelerator (E) used in the present invention include amines, sulfinic acid and salts thereof, borate compounds, derivatives of barbituric acid, triazine compounds, copper compounds, tin compounds, vanadium compounds, halogen compounds, aldehydes, thiol compounds, sulfites, bisulfites, and thiourea compounds.

The amines used as polymerization accelerator (E) can be categorized into aliphatic amines and aromatic amines. Examples of the aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. In view of the curability and storage stability of the dental composition, preferred for use are tertiary aliphatic amines, and N-methyldiethanolamine and triethanolamine are more preferred.

Examples of the aromatic amines include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N, N-bis(2-hydroxyethyl)-3,5-diisopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, propyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of the ability to impart superior curability to the dental composition, it is preferable to use at least one selected from the group consisting of N,N-bis(2-hydroxyethyl)-p-toluidine, ethyl 4-(N,N-dimethylamino)benzoate, n-butoxyethyl 4-(N,N-dimethylamino)benzoate, and 4-(N,N-dimethylamino)benzophenone.

Specific examples of sulfinic acid and salts thereof, borate compounds, derivatives of barbituric acid, triazine compounds, copper compounds, tin compounds, vanadium compounds, halogen compounds, aldehydes, thiol compounds, sulfites, bisulfites, and thiourea compounds include those mentioned in WO2008/087977.

The polymerization accelerator (E) may be contained alone, or two or more thereof may be contained in combination. The content of the polymerization accelerator (E) used in the present invention is not particularly limited. However, in view of curability and other properties of the dental composition obtained, the content of polymerization accelerator (E) is preferably 0.001 parts or more by mass, more preferably 0.01 parts or more by mass, even more preferably 0.1 parts or more by mass, and is preferably 30 parts or less by mass, more preferably 10 parts or less by mass, even more preferably 5 parts or less by mass with respect to total 100 parts by mass of the compound (A) and the monomer (B) in the dental composition. When the content of polymerization accelerator (E) is less than 0.001 parts by mass, polymerization may fail to proceed sufficiently, and this may lead to a decrease of adhesive properties. In this respect, the content of polymerization accelerator (E) is more preferably 0.05 parts or more by mass. When the content of polymerization accelerator (E) is more than 30 parts by mass, it may not be possible to obtain sufficient adhesive properties when the polymerization initiator itself has low polymerization performance, in addition to raising a possibility of precipitation from the dental composition. In this respect, the content of polymerization accelerator (E) is more preferably 20 parts or less by mass.

[Fluorine-Ion Releasing Substance]

A dental composition of the present invention may further comprise a fluorine-ion releasing substance. By containing a fluorine-ion releasing substance, the dental composition produced can impart acid resistance to tooth structure. Examples of the fluorine-ion releasing substance include metal fluorides such as sodium fluoride, potassium fluoride, sodium monofluorophosphate, lithium fluoride, and ytterbium fluoride. The fluorine-ion releasing substance may be contained alone, or two or more thereof may be contained in combination.

The dental composition may additionally comprise additives such as pH adjusters, polymerization inhibitors, thickeners, colorants, fluorescent agents, fragrances, and cross-linking agents (for example, polyvalent metal ion releasing components), provided that such additives do not hinder the effects of the present invention. These may be used alone, or two or more thereof may be used in combination. A dental composition of the present invention may also comprise anti-microbial substances such as cetylpyridinium chloride, benzalkonium chloride, (meth)acryloyloxydodecylpyridinium bromide, (meth)acryloyloxyhexadecylpyridinium chloride, (meth)acryloyloxydecylammonium chloride, and triclosan. A dental composition of the present invention may comprise a known dye or pigment as a colorant.

A dental composition of the present invention may comprise a solvent, depending on use. The solvent may be, for example, water or an organic solvent. The organic solvent may be any known organic solvent. Examples of such

solvents include alcohol solvents (such as methanol, ethanol, 1-propanol, and 2-propanol), acetone, methyl ethyl ketone, tetrahydrofuran, diethyl ether, diisopropyl ether, hexane, toluene, chloroform, ethyl acetate, and butyl acetate. Preferred are alcohol solvents. In an embodiment using an organic solvent, the content of organic solvent is preferably 1 to 2,000 parts by mass, more preferably 2 to 1,000 parts by mass, even more preferably 3 to 500 parts by mass with respect to total 100 parts by mass of the compound (A) and the monomer (B). For example, when used as a self-adhesive dental composite resin, a dental composite resin, or a dental cement, a dental composition of the present invention may be a dental composition that does not comprise a solvent. However, inclusion of trace amounts of water or organic solvent (for example, 3 mass% or less with respect to the composition) is acceptable, provided that it does not cause problems such as improper curing or delayed cure. Some commercially available products are sold as products containing water or organic solvent (for example, colloidal silica), depending on the components. Such products can be used for the preparation of a dental composition of the present invention after removing water or organic solvent to the acceptable limits.

The components (for example, a prepolymer (oligomer) different from compound (A)) of a dental composition of the present invention other than the compound (A), the monomer (B), the polymerization initiator (C), the filler (D), the polymerization accelerator (E), and polymerization inhibitors and colorants are preferably less than 0.1 parts by mass, more preferably less than 0.01 parts by mass, even more preferably less than 0.001 parts by mass in 100 parts by mass of the dental composition. A dental composition of the present invention is preferably one having a polymerization shrinkage stress of less than 10 MPa, more preferably less than 9.5 MPa, even more preferably less than 9.0 MPa. The method of measurement of polymerization shrinkage stress is as described in the EXAMPLES section below.

A dental composition of the present invention can be used for dental treatment as, for example, a dental composite resin (particularly preferably, a self-adhesive dental composite resin), a dental bonding material, a dental cement, a pit and fissure sealant, a loose tooth fixing material, an abutment construction material, or an orthodontic bonding material. Particularly, a dental composition of the present invention is suited as a self-adhesive dental composite resin, a dental composite resin, or a dental cement. In such applications, a dental composition of the present invention may be provided in two bottles or two pastes of divided components, or may be provided in one bottle or one paste. The following describes specific embodiments of different uses of a dental composition of the present invention.

<Self-Adhesive Dental Composite Resin>

A preferred embodiment of a dental composition of the present invention is, for example, a self-adhesive dental composite resin. A self-adhesive dental composite resin formed of a dental composition of the present invention comprises a monomer (B-1) having an acidic group. When a dental composition of the present invention is used as a self-adhesive dental composite resin, it is preferable that a dental composition of the present invention comprise a compound (A), a monomer (B), a polymerization initiator (C), a filler (D), and a polymerization accelerator (E), and that the monomer (B) comprise a monomer (B-1) having an acidic group, a hydrophobic monomer (B-2) having no acidic group, and a hydrophilic monomer (B-3) having no acidic group. The polymerization initiator (C) is preferably a photopolymerization initiator. More preferably, the polymerization initiator (C) comprises a water-soluble photopolymerization initiator (C-1) and a water-insoluble photopolymerization initiator (C-2). A pretreatment agent may be used when a dental composition of the present invention is used as a self-adhesive dental composite resin. However, because a self-adhesive dental composite resin has self-adhesive properties, a pretreatment agent is not essential, and it is not necessarily required to use a pretreatment agent. In this way, a dental composition of the present invention can be solely used as a self-adhesive dental composite resin, with no pretreatment agent.

The content of each component in the self-adhesive dental composite resin is such that the dental composition comprises preferably 0.1 to 50 parts by mass of compound (A), 1 to 50 parts by mass of monomer (B-1) having an acidic group, 20 to 99 parts by mass of hydrophobic monomer (B-2) having no acidic group, and 0 to 50 parts by mass of hydrophilic monomer (B-3) having no acidic group, more preferably 0.5 to 40 parts by mass of compound (A), 1 to 40 parts by mass of monomer (B-1) having an acidic group, 40 to 99 parts by mass of hydrophobic monomer (B-2) having no acidic group, and 0 to 40 parts by mass of hydrophilic monomer (B-3) having no acidic group, even more preferably 1 to 35 parts by mass of compound (A), 1 to 30 parts by mass of monomer (B-1) having an acidic group, 60 to 99 parts by mass of hydrophobic monomer (B-2) having no acidic group, and 0 to 30 parts by mass of hydrophilic monomer (B-3) having no acidic group with respect to total 100 parts by mass of the compound (A) and the monomer (B) in the dental composition. The dental composition preferably comprises 0.001 to 30 parts by mass of polymerization initiator (C), 50 to 2,000 parts by mass of filler (D), and 0.001 to 20 parts by mass of polymerization accelerator (E), and more preferably comprises 0.05 to 10 parts by mass of polymerization initiator (C), 100 to 1,500 parts by mass of filler (D), and 0.05 to 10 parts by mass of polymerization accelerator (E) with respect to total 100 parts by mass of the compound (A) and the monomer (B). The dental composition may or may not contain a hydrophilic monomer (B-3) when used as a self-adhesive dental composite resin.

<Dental Composite Resin (Excluding Self-Adhesive Dental Composite Resin)>

Another preferred embodiment of a dental composition of the present invention is, for example, a dental composite resin. A dental composite resin formed of a dental composition of the present invention does not comprise a monomer (B-1) having an acidic group. When used as a dental composite resin, a dental composition of the present invention preferably comprises a compound (A), a hydrophobic monomer (B-2) having no acidic group, a hydrophilic monomer (B-3) having no acidic group, a polymerization initiator (C), a filler (D), and a polymerization accelerator (E). The polymerization initiator (C) preferably comprises a photopolymerization initiator. More preferably, the polymerization initiator (C) comprises a water-soluble photopolymerization initiator (C-1) and a water-insoluble photopolymerization initiator (C-2). When a dental composition of the present invention is used as a dental composite resin, the use of a dental bonding material or a pretreatment agent is essential.

The content of each component in the dental composite resin is such that the dental composition comprises preferably 0.1 to 50 parts by mass of compound (A), 50 to 99 parts by mass of hydrophobic monomer (B-2) having no acidic group, and 0 to 40 parts by mass of hydrophilic monomer (B-3) having no acidic group, more preferably 0.5 to 40 parts by mass of compound (A), 60 to 99 parts by mass of hydrophobic monomer (B-2) having no acidic group, and 0 to 30 parts by mass of hydrophilic monomer (B-3) having no acidic group, even more preferably 1 to 35 parts by mass of compound (A), 70 to 99 parts by mass of hydrophobic monomer (B-2) having no acidic group, and 0 to 20 parts by mass of hydrophilic monomer (B-3) having no acidic group with respect to total 100 parts by mass of the compound (A) and the monomer (B) in the dental composition. The dental composition comprises preferably 0.001 to 30 parts by mass of polymerization initiator (C), 50 to 2,000 parts by mass of filler (D), and 0.001 to 20 parts by mass of polymerization accelerator (E), more preferably 0.05 to 10 parts by mass of polymerization initiator (C), 100 to 1,500 parts by mass of filler (D), and 0.05 to 10 parts by mass of polymerization accelerator (E) with respect to total 100 parts by mass of the compound (A) and the monomer (B). The dental composition may or may not contain a hydrophilic monomer (B-3) when used as a dental composite resin.

<Dental Cement>

Another preferred embodiment of a dental composition of the present invention is, for example, a dental cement. Preferred examples of the dental cement include resin cements, glass ionomer cements, and resin-reinforced glass ionomer cements. The dental cement may use, for example, a self-etching primer as a pretreatment agent. When used as a dental cement, it is preferable that a dental composition of the present invention comprise a compound (A), a monomer (B), a polymerization initiator (C), a filler (D), and a polymerization accelerator (E), and that the monomer (B) comprise a monomer (B-1) having an acidic group, a hydrophobic monomer (B-2) having no acidic group, and a hydrophilic monomer (B-3) having no acidic group. The polymerization initiator (C) preferably comprises a chemical polymerization initiator. More preferably, a chemical polymerization initiator and a photopolymerization initiator are used in combination. Preferably, the photopolymerization initiator is a combination of a water-soluble photopolymerization initiator (C-1) and a water-insoluble photopolymerization initiator (C-2).

The content of each component in the dental cement is such that the dental composition comprises preferably 0.1 to 50 parts by mass of compound (A), 0 to 50 parts by mass of monomer (B-1) having an acidic group, 50 to 99 parts by mass of hydrophobic monomer (B-2) having no acidic group, and 0 to 50 parts by mass of hydrophilic monomer (B-3) having no acidic group, more preferably 0.5 to 40 parts by mass of compound (A), 0 to 40 parts by mass of monomer (B-1) having an acidic group, 60 to 99 parts by mass of hydrophobic monomer (B-2) having no acidic group, and 0 to 40 parts by mass of hydrophilic monomer (B-3) having no acidic group, even more preferably 1 to 35 parts by mass of compound (A), 0 to 30 parts by mass of monomer (B-1) having an acidic group, 70 to 99 parts by mass of hydrophobic monomer (B-2) having no acidic group, and 0 to 30 parts by mass of hydrophilic monomer (B-3) having no acidic group with respect to total 100 parts by mass of the compound (A) and the monomer (B) in the dental composition. The dental composition comprises preferably 0.001 to 30 parts by mass of polymerization initiator (C), 50 to 2,000 parts by mass of filler (D), and 0.001 to 20 parts by mass of polymerization accelerator (E), more preferably 0.05 to 10 parts by mass of polymerization initiator (C), 100 to 1,500 parts by mass of filler (D), and 0.05 to 10 parts by mass of polymerization accelerator (E) with respect to total 100 parts by mass of the compound (A) and the monomer (B). When used as a dental cement, the dental composition may or may not contain a hydrophilic monomer (B-3). The dental composition is not required to contain a monomer (B-1) having an acidic group when the dental composition is used as a dental cement of a type that uses a pretreatment agent.

In all of these preferred embodiments as a self-adhesive dental composite resin, a dental composite resin, and a dental cement, the content of each component may be appropriately varied, and changes such as addition and deletion of any component may be made following the above descriptions of the present specification.

The present invention encompasses embodiments combining the foregoing features, provided that the present invention can exhibit its effects with such combinations made in various forms within the technical idea of the present

invention.

EXAMPLES

The following describes the present invention in greater detail by way of Examples. However, the present invention is not limited by the following EXAMPLES. It should also be noted that the combinations of the features described in the EXAMPLES below do not necessarily represent all the means necessary for solving the problems identified in the present invention. The components used in the following Examples and Comparative Examples, and the abbreviations and the structures of these components are presented below, along with the test methods used.

[Compound (A)]

UN-7600: urethane acrylate (manufactured by Negami Chemical Industrial Co., Ltd.; viscosity: 1,100,000 cps/25°C; weight-average molecular weight (Mw): 11,500; glass transition temperature (Tg): -42°C and 44.6°C; polyester skeleton-containing urethane acrylate; number of polymerizable groups (acryl groups): 2; weight-average molecular weight per polymerizable group: 5,750; acetone solubility: less than 10 g/L)
UC-102M: polyisoprene (manufactured by Kuraray Co., Ltd.; viscosity: 30,000 cps (38°C); weight-average molecular weight (Mw): 17,000; glass transition temperature (Tg): -60°C; number of polymerizable groups (methacryl groups): 2; weight-average molecular weight per polymerizable group: 8,500; acetone solubility: less than 10 g/L)
TE-2000: urethane methacrylate (unhydrogenated) (manufactured by Nippon Soda Co., Ltd.; viscosity: 1,500,000 cps/45°C; weight-average molecular weight (Mw): about 2,000; glass transition temperature: -9°C; polybutadiene skeleton-containing urethane methacrylate; number of polymerizable groups (methacryl groups): about 1.7; weight-average molecular weight per polymerizable group: about 1,250 or more; acetone solubility: less than 10 g/L)

[Compound with Weight-Average Molecular Weight of 1,000 or More]

UN-2600: urethane acrylate (manufactured by Negami Chemical Industrial Co., Ltd.; viscosity: 75,000 to 90,000 cps/25°C; weight-average molecular weight (Mw): 2,500; glass transition temperature (Tg): -1°C; number of polymerizable groups (acryl groups): 2; weight-average molecular weight per acryl group: 1,250; acetone solubility: 50 g/L or more)

[Monomer (B)]

MDP: 10-methacryloyloxydecyl dihydrogen phosphate
D-2.6E: 2,2-bis(4-methacryloyloxypolyethoxyphenyl)propane (average number of moles of ethoxy group added: 2.6)
3G: triethylene glycol dimethacrylate
DD: 1,10-decanediol dimethacrylate
MAEA: N-methacryloyloxyethylacrylamide
HEMA: 2-hydroxyethyl methacrylate

The acetone solubilities of the mixtures of the monomer (B) components are presented in Tables 1 and 2.

[Polymerization Initiator (C)]

· Water-soluble photopolymerization initiator (C-1)

Li-TPO: a lithium salt of phenyl(2,4,6-trimethylbenzoyl)phosphinic acid (a compound represented by the following formula (3)); average particle diameter: 5 μm

[Chem. 3]

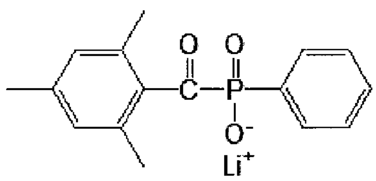

(3)

· Water-insoluble photopolymerization initiator (C-2)

CQ: dl-camphorquinone

[Filler (D)]

Inorganic filler 1: particulate silica Aerosil® R 972 manufactured by Nippon Aerosil Co., Ltd., average particle diameter: 16 nm, refractive index: 1.46
Inorganic filler 2: silane-treated silica stone powder; refractive index: 1.55

A silica stone powder (quartz, Hi-Silica manufactured by Nitchitsu Co., Ltd. under this trade name) was pulverized with a ball mill to obtain a pulverized silica stone powder. The pulverized silica stone powder had an average particle diameter of 2.2 μm when measured by volume with a laser diffraction particle size distribution analyzer (Model SALD-2300, manufactured by Shimadzu Corporation). The silane-treated silica stone powder was obtained after the surface treatment of 100 parts by mass of pulverized silica stone powder with 4 parts by mass of γ-methacryloyloxypropyltrimethoxysilane, using an ordinary method.

[Polymerization Accelerator (E)]

DABE: ethyl 4-(N,N-dimethylamino)benzoate

[Other]

BHT: 2,6-di-t-butyl-4-methylphenol (a stabilizer, a polymerization inhibitor)

[Application of Dental Composition to Self-Adhesive Dental Composite Resin or Dental Composite Resin]

<Examples 1-1 to 1-10 and Comparative Examples 1-1 to 1-7>

The above components were used to prepare pastes. Specifically, the components shown in Tables 1 and 2 were mixed and kneaded at ordinary temperature to prepare pastes (compositions) of the self-adhesive dental composite resins of Examples 1-1 to 1-9, the dental composite resin of Example 1-10, the self-adhesive dental composite resins of Comparative Examples 1-1 to 1-5, and the dental composite resins of Comparative Examples 1-6 and 1-7. These pastes were measured for polymerization shrinkage stress and flexural properties using the methods described below. Tables 1 and 2 show the proportion (parts by mass) of each component in the dental composite resins of Examples and Comparative Examples, along with the test results.

[Measurement of Glass Transition Temperature]

The compound (A) and UN-2600 were separately dissolved in hexane with CQ and DABE to obtain specimens. Specifically, CQ and DABE were dissolved in hexane, 1 mass% each with respect to the compound (A) and UN-2600, and the hexane was distilled away to obtain a specimen. The specimen was held between two glass slides, and the glass slides were pressed against the specimen with metal spacers until the specimen had a thickness of 500 μm. The specimen was then cured into a cured product by exposing the glass slides to light from both sides, 3 minutes on each side, using a dental LED photoirradiator (alpha-Light V manufactured by J. Morita Corp. under this trade name). Immediately after cure, the cured product was cut into 2.3 to 2.5 mg with a razor to obtain measurement samples. The measurement sample was then filled into the sample pan of a heat flux DSC measurement device (DSC 214 Polyma manufactured by NETZSCH Japan under this trade name). In the heat flux DSC measurement device, the sample was isothermally heated at -150°C for 5 minutes, and the temperature was increased to 200°C at a rate of temperature increase of 20°C/min in a 40 mL/min nitrogen gas atmosphere to measure glass transition temperature (n = 3). The mean value was determined as the glass transition temperature.

[Acetone Solubility]

A sample (0.5 g) was weighed into a 50 mL Erlenmeyer flask equipped with a magnetic stirrer, and was stirred at room temperature for 30 minutes after adding 10 mL of acetone. The sample was determined as having dissolved

when there was no visually observable undissolved substance, and the solution appeared transparent. For samples that did not dissolve, the same procedure was repeated with decreasing sample amounts, ending with a final addition of 0.1 g of sample.

[Measurement of Polymerization Shrinkage Stress]

A stainless-steel washer (5.3 mm in inner diameter × 0.8 mm in thickness) after the application of a release agent was installed on a 5.0 mm thick glass plate that had been subjected to sandblasting with a 50 μm alumina powder. The paste of the dental composite resin of each Example and Comparative Example was then filled into the washer. After removing the excess paste, the dental composite resin paste was held between the glass plate and a stainless-steel jig (∅ = 5 mm) that had been separately subjected to sandblasting.

The paste was subjected to 10 seconds of photoirradiation from the glass plate side to cure the dental composite resin, using a dental LED photoirradiator (PenCure 2000 manufactured by J. Morita Corp. under this trade name). The resulting polymerization shrinkage stress was measured with a universal testing machine (Autograph AG-I 100kN, manufactured by Shimadzu Corporation) (n = 3), and the mean value was calculated.

[Evaluation of Flexural Properties]

The strength was evaluated by conducting a flexure test in compliance with ISO 4049:2009, specifically as follows. The dental composite resin paste was filled into a SUS die (2 mm in length × 25 mm in width × 2 mm in thickness), and glass slides were pressed against the paste (dental composition) from the top and bottom (over a 2 mm × 25 mm surface). The dental composition was cured by applying light through the glass slides from both sides, 5 points on each side, 10 seconds each, using a dental LED photoirradiator (PenCure 2000 manufactured by J. Morita Corp.). The cured product was measured for three-point flexural strength and flexural modulus by conducting a flexure test at a span length of 20 mm and a crosshead speed of 1 mm/min, using a universal testing machine (Autograph AG-I, 100kN, manufactured by Shimadzu Corporation) (n = 5), and the mean value was calculated.

[Table 1]

| Components (parts by mass) | | Ex. 1-1 | Ex. 1-2 | Ex. 1-3 | Ex. 1-4 | Ex. 1-5 | Ex. 1-6 | Ex. 1-7 | Ex. 1-8 | Ex. 1-9 | Ex. 1-10 |
|---|---|---|---|---|---|---|---|---|---|---|---|
| Compound (A) | UN-7600 | 10 | 5 | 15 | 20 | 30 | - | - | 15 | 5 | 10 |
| | TE-2000 | - | - | - | - | - | 10 | - | - | - | - |
| | UC-102M | - | - | - | - | - | - | 10 | - | - | - |
| Compound having a weight-average molecular weight of 1,000 or more other than (A) | UN-2600 | - | - | - | - | - | - | - | - | - | - |
| Monomer (B-1) having an acidic group | MDP | 10 | 10 | 10 | 10 | 5 | 10 | 10 | 10 | 10 | - |
| Hydrophobic monomer (B-2) having no acidic group | D-2.6E | 50 | 55 | 45 | 40 | 40 | 50 | 50 | 45 | 55 | 70 |
| | DD | 20 | 20 | 20 | 20 | 20 | 20 | 20 | 10 | - | 20 |
| | 3G | - | - | - | - | - | - | - | 10 | - | - |
| | MAEA | 10 | 10 | 10 | 10 | 5 | 10 | 10 | 10 | 10 | - |
| Hydrophilic monomer (B-3) having no acidic group | HEMA | - | - | - | - | - | - | - | - | 20 | - |
| Polymerization initiator (C) | CQ | 0.2 | 0.2 | 0.2 | 0.2 | 0.2 | 0.2 | 0.2 | 0.2 | 0.2 | 0.3 |
| | Li-TPO | 1 | 1 | 1 | 1 | 1 | 1 | 1 | 1 | 1 | - |
| Filler (D) | Inorganic filler 1 | 10 | 10 | 10 | 10 | 10 | 10 | 10 | 10 | 10 | 10 |
| | Inorganic filler 2 | 170 | 170 | 170 | 170 | 170 | 170 | 170 | 170 | 170 | 170 |
| Polymerization accelerator (E) | DABE | 0.2 | 0.2 | 0.2 | 0.2 | 0.2 | 0.4 | 0.4 | 0.4 | 0.4 | 0.4 |
| Other | BHT | 0.05 | 0.05 | 0.05 | 0.05 | 0.05 | 0.05 | 0.05 | 0.05 | 0.05 | 0.05 |
| Acetone solubility of monomer (B) | g/L | 50 or more | 50 or more | 50 or more | 50 or more | 50 or more | 50 or more | 50 or more | 50 or more | 50 or more | 50 or more |
| Polymerization shrinkage stress | MPa | 7.8 | 8.5 | 7.2 | 6.4 | 5.9 | 8.2 | 8.4 | 8.3 | 7.8 | 6.6 |
| Flexural modulus | GPa | 6.2 | 6.5 | 6.1 | 5.7 | 5.3 | 5.9 | 4.4 | 5.9 | 4.8 | 4.6 |
| Flexural strength | MPa | 105 | 108 | 100 | 95 | 82 | 110 | 104 | 97 | 99 | 100 |

[Table 2]

| Components (parts by mass) | | Com. Ex. 1-1 | Com. Ex. 1-2 | Com. Ex. 1-3 | Com. Ex. 1-4 | Com. Ex. 1-5 | Com. Ex. 1-6 | Com. Ex. 1-7 |
|---|---|---|---|---|---|---|---|---|
| Compound (A) | UN-7600 | - | - | - | - | - | - | - |
| | UC-102M | - | - | - | - | - | - | - |
| | TE-2000 | - | - | - | - | - | - | - |
| Compound having a weight-average molecular weight of 1,000 or more other than (A) | UN-2600 | - | - | - | 10 | 20 | - | 10 |
| Monomer (B-1) having an acidic group | MDP | 10 | 10 | 10 | 10 | 10 | - | - |
| Hydrophobic monomer (B-2) having no acidic group | D-2.6E | 60 | 60 | 60 | 60 | 50 | 75 | 70 |
| | DD | 20 | - | - | 10 | 10 | 25 | 20 |
| | 3G | - | 20 | - | - | - | - | - |
| | MAEA | 10 | 10 | 10 | 10 | 10 | - | - |
| Hydrophilic monomer (B-3) having no acidic group | HEMA | - | - | 20 | - | - | - | - |
| Polymerization initiator (C) | CQ | 0.2 | 0.2 | 0.2 | 0.2 | 0.2 | 0.3 | 0.3 |
| | Li-TPO | 1 | 1 | 1 | 1 | 1 | - | - |
| Filler (D) | Inorganic filler 1 | 10 | 10 | 10 | 10 | 10 | 10 | 10 |
| | Inorganic filler 2 | 170 | 170 | 170 | 170 | 170 | 170 | 170 |
| Polymerization accelerator (E) | DABE | 0.2 | 0.2 | 0.2 | 0.2 | 0.2 | 0.4 | 0.4 |
| Other | BHT | 0.05 | 0.05 | 0.05 | 0.05 | 0.05 | 0.05 | 0.05 |
| Acetone solubility of monomer (B) | g/L | 50 or more | 50 or more | 50 or more | 50 or more | 50 or more | 50 or more | 50 or more |
| Polymerization shrinkage stress | MPa | 10.5 | 12.0 | 10.4 | 9.5 | 9.3 | 9.5 | 9.0 |
| Flexural modulus | GPa | 6.6 | 6.8 | 5.3 | 5.3 | 4.6 | 6.2 | 4.6 |
| Flexural strength | MPa | 110 | 121 | 99 | 104 | 110 | 114 | 109 |

As can be seen in Tables 1 and 2, the self-adhesive dental composite resins according to the present invention (Examples 1-1 to 1-9) produced cured products having a flexural strength of 82 MPa or more, a value sufficient for practical applications, and the cured products exhibited a low polymerization shrinkage stress of 8.5 MPa or less. The dental composite resin according to the present invention (Example 1-10) produced a cured product having a flexural strength of 100 MPa, and the cured product exhibited a low polymerization shrinkage stress of 6.6 MPa. In contrast, the polymerization shrinkage stress was 9.3 MPa or more in the self-adhesive dental composite resins (Comparative Examples 1-1 to 1-5) that did not contain a compound (A), or that contained a compound having a weight-average molecular weight of 1,000 or more other than (A), as shown in Table 2. The polymerization shrinkage stress was 9.0 MPa or more in the self-adhesive dental composite resins (Comparative Examples 1-6 and 1-7) that did not contain a compound (A), or that contained a compound having a weight-average molecular weight of 1,000 or more other than (A). As demonstrated above, the reduction in polymerization shrinkage stress was found to be insufficient in the comparative examples. These results suggest that containing a compound (A) can achieve relaxation of polymerization shrinkage stress, and can effectively reduce the risk of detachment or marginal leakage in the restorative treatment of relatively deep cavities.

INDUSTRIAL APPLICABILITY

A dental composition according to the present invention is suited for use as a dental composite resin, a self-adhesive dental composite resin, or a dental cement in the field of dental treatment.

## Claims

1. A dental composition comprising a compound (A), a monomer (B) having a solubility of 50 g/L or more in acetone at 25°C, and a polymerization initiator (C),
wherein the compound (A) has a weight-average molecular weight of 1,000 to 80,000, and includes a Tg of less than 40°C, in addition to having a solubility of less than 50 g/L in acetone at 25°C.

2. The dental composition according to claim 1, wherein the compound (A) comprises a compound having a polymerizable group.

3. The dental composition according to claim 1 or 2, wherein the content of the compound (A) is 0.1 to 50 parts by mass with respect to total 100 parts by mass of the compound (A) and the monomer (B).

4. The dental composition according to claim 2 or 3, wherein the compound having a polymerizable group comprises a urethanized (meth)acrylic compound (A-1) having a urethane bond.

5. The dental composition according to any one of claims 1 to 4, wherein the compound (A) has a solubility of less than 20 g/L in acetone at 25°C.

6. The dental composition according to any one of claims 1 to 5, wherein the monomer (B) comprises a monomer (B-1) having an acidic group.

7. The dental composition according to any one of claims 1 to 6, which further comprises a filler (D).

8. The dental composition according to any one of claims 2 to 7, wherein the compound (A) has a weight-average molecular weight of 1,250 or more and less than 20,000 per polymerizable group.

9. A dental composite resin comprising a dental composition of any one of claims 1 to 8.

10. A self-adhesive dental composite resin comprising a dental composition of any one of claims 1 to 8.

11. A dental cement comprising a dental composition of any one of claims 1 to 8.

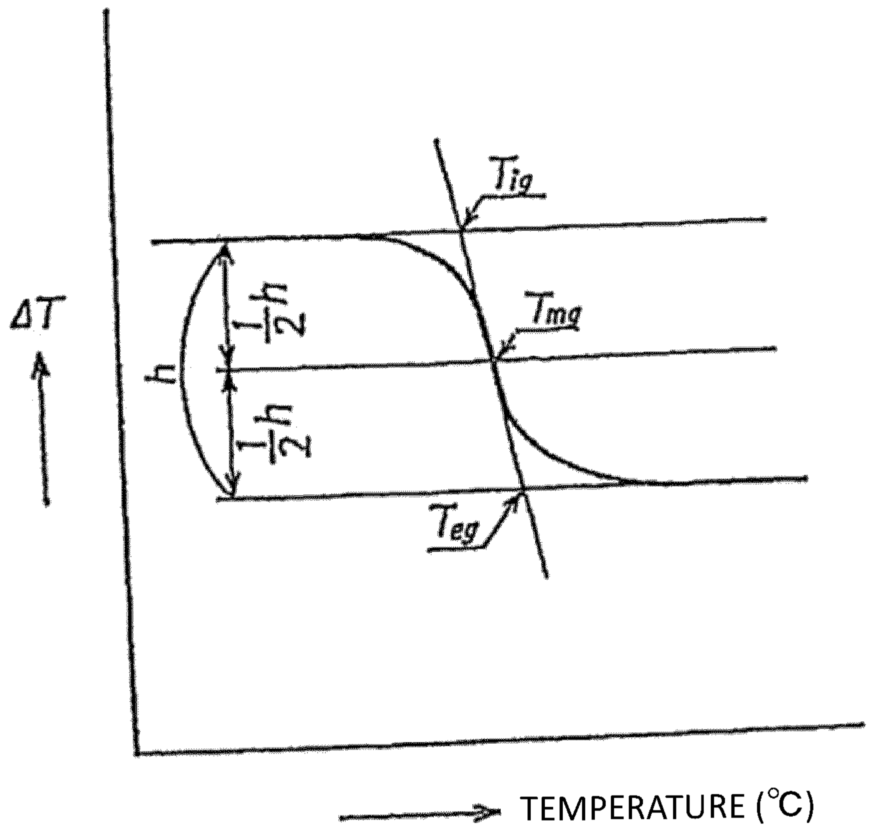


FIG.1

## INTERNATIONAL SEARCH REPORT

International application No.
**PCT/JP2021/048424**

**A. CLASSIFICATION OF SUBJECT MATTER**

***A61K 6/887***(2020.01)i; ***A61C 13/15***(2006.01)i
FI: A61K6/887; A61C13/15

According to International Patent Classification (IPC) or to both national classification and IPC

**B. FIELDS SEARCHED**

Minimum documentation searched (classification system followed by classification symbols)

A61K6/887; A61C13/15

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/MEDLINE/EMBASE/BIOSIS (STN), JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | JP 2011-144121 A (TOKUYAMA DENTAL CORP) 28 July 2011 (2011-07-28)<br>entire text, in particular, claims, paragraphs [0014]-[0018], examples | 1-11 |
| A | JP 2003-512403 A (KERR CORPORATION) 02 April 2003 (2003-04-02)<br>entire text, in particular, claims, examples | 1-11 |
| A | WO 2020/122192 A1 (KURARAY NORITAKE DENTAL INC) 18 June 2020 (2020-06-18)<br>entire text, in particular, claims, paragraphs [0057]-[0059], [0160], examples | 1-11 |
| A | 川口稔, 歯科用新レジンの開発に関する研究　ーウレタンジメタクリレートの構造と物性についてー, 歯科材料・器械, 1988, vol. 7, no. 2, pp. 143-158, ISSN: 0286-5858, (KAWAGUCHI, Minoru. Development of New Dental Resin Materials - Relationship between the Structure and Physical Properties of Urethane Dimethacrylate Polymers -. The Journal of the Japanese Society for Dental Materials and Devices.)<br>entire text | 1-11 |
| P, X | WO 2021/070875 A1 (KURARAY NORITAKE DENTAL INC) 15 April 2021 (2021-04-15)<br>in particular, examples 1-4 | 1-11 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **07 February 2022** | **22 February 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

**INTERNATIONAL SEARCH REPORT**
**Information on patent family members**

International application No.
**PCT/JP2021/048424**

| Patent document cited in search report | Publication date (day/month/year) | Patent family member(s) | Publication date (day/month/year) |
|---|---|---|---|
| JP 2011-144121 A | 28 July 2011 | (Family: none) | |
| JP 2003-512403 A | 02 April 2003 | WO 2001/030302 A1<br>claims, examples<br>US 6353041 B1<br>US 6472454 B1<br>EP 1221928 A1<br>AU 2115301 A<br>BR 14794 A<br>CN 1399535 A<br>MX PA02003880 A | |
| WO 2020/122192 A1 | 18 June 2020 | (Family: none) | |
| WO 2021/070875 A1 | 15 April 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014024775 A
- JP 2012188672 A
- JP 2002256010 A
- JP H1129428 A
- JP 2016175851 A
- JP 50042696 A
- JP 2006510583 T
- JP 2009184971 A
- JP 2011144121 A
- JP 100 A
- JP 200 A
- JP S57197289 A
- WO 2014095724 A
- JP H93109 A
- JP H10245525 A
- WO 2008087977 A


### Non-patent literature cited in the description


- **SUH, CLARKE.** *J. P. S.,* 1967, vol. A-1 (5), 1671-1681
- **FEDORS et al.** Specifically, the SP value can be determined by referring to Polymer Engineering and Science. ROBERF. FEDORS, February 1974, vol. 14, 147-154